Europäisches Patentamt
European Patent Office
Office européen des brevets

(11) Veröffentlichungsnummer: **0 435 127 A2**

# EUROPÄISCHE PATENTANMELDUNG

(21) Anmeldenummer: **90124485.5**

(22) Anmeldetag: **18.12.90**

(51) Int. Cl.$^5$: **C07D 213/30**, A01N 43/40, C07D 405/04, A01N 43/90

(30) Priorität: **29.12.89 DE 3943277**
**28.08.90 DE 4027139**

(43) Veröffentlichungstag der Anmeldung:
**03.07.91 Patentblatt 91/27**

(84) Benannte Vertragsstaaten:
**AT BE CH DE DK ES FR GB GR IT LI NL SE**

(71) Anmelder: **BASF Aktiengesellschaft**
**Carl-Bosch-Strasse 38**
**W-6700 Ludwigshafen(DE)**

(72) Erfinder: **Zierke, Thomas, Dr.**
**Bahnhofstrasse 10**
**W-6737 Boehl-Iggelheim(DE)**
Erfinder: **Zipperer, Bernhard, Dr.**
**Am Herrgottsacker 6**
**W-6716 Dirmstein(DE)**
Erfinder: **Sauter, Hubert, Dr.**
**Neckarpromenade 20**
**W-6800 Mannheim 1(DE)**
Erfinder: **Ammermann, Eberhard, Dr.**
**Sachsenstrasse 3**
**W-6700 Ludwigshafen(DE)**
Erfinder: **Lorenz, Gisela, Dr.**
**Erlenweg 13**
**W-6730 Neustadt(DE)**

(54) **3-Substituierte Pyridine.**

(57) 3-substituierte Pyridine I

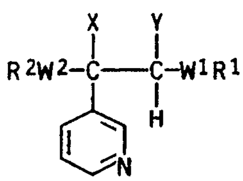

I

(X = OH; Y = -O-CHO, -O-CO-$W^3R^3$, -O-$SO_2$-$W^3R^3$ oder X+Y = -O-CH($W^3R^3$)-O-, $^3O^3CH(O^3W^4R^4)$-O-, -O-C-($W^3R^3$)(O-$W^4R^4$)-O-; $W^1$-$W^4$ = direkte Bindung, -$CH_2$-, -$CH_2$-$CH_2$, -CH($CH_3$)-, -$CH_2O$- -$CH_2S$-; $R^1$-$R^4$ = gegebenenfalls durch Cycloalkyl subst. $C_1$-$C_6$-Alkyl, $C_1$-$C_6$-Halogenalkyl, gegebenenfalls durch 1-3 Alkylreste subst. $C_3$-$C_8$-Cycloalkyl, $C_1$-$C_4$-Alkoxy-$C_1$-$C_4$-alkyl, $C_1$-$C_4$-Alkylthio-$C_1$-$C_4$-alkyl, $C_2$-$C_6$-Alkenyl, $C_2$-$C_6$-Alkinyl, gegebenenfalls subst. Phenyl oder Naphthyl, gegebenenfalls subst. 5-/6-gliedriges Heteroaryl mit 1 bis 3 Heteroatomen;
$R^2$ zusätzlich H, wenn X+Y= -O-CH($W^3R^3$)-O-, -O-CH(O-$W^4R^4$)-O- oder -O-C($W^3R^3$)(O-$W^4R^4$)-O- und W2 = direkte Bindung)
ausgenommen cis-1-(2,4-Dichlorphenyl)-2-(pyrid-3-yl)-1,2-epoxypropan, trans-1-(2,4-Dichlorphenyl)-2-(pyrid-3-yl)-1,2-epoxypropan, 1-(2,4-Dichlorphenyl)-2-(pyrid-3-yl)-1,2-epoxybutan und Methansulfonsäure-2,4-dichlor-α-[1-hydroxy-1-(pyrid-3-yl)-ethyl]-benzylester,
sowie die N-Oxide und die pflanzenverträglichen mineralsauren Salze und Metallkomplexe von I.
Die Verbindungen I eignen sich als Fungizide.

EP 0 435 127 A2

# 3-SUBSTITUIERTE PYRIDINE

Die vorliegende Erfindung betrifft neue 3-substituierte Pyridine der Formel I

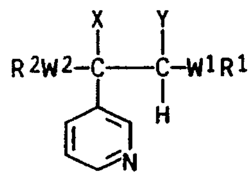

I

in der die Variablen die folgende Bedeutung haben:

X

die Hydroxylgruppe und

Y

einen Rest -O-CHO, -O-CO-$W^3R^3$ oder -O-$SO_2$-$W^3R^3$ oder

X und Y

zusammen Sauerstoff oder eine Gruppe

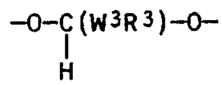 , 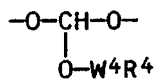 oder 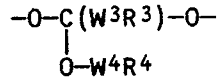 ;

$W^1$-$W^4$

eine direkte Bindung oder eine Methylen-, Ethylen-, Methyl-methylen-, Methylenoxy- oder Methylenthiogruppe, wobei die Verknüpfung der beiden letzten Gruppen mit den Resten $R^1$ bis $R^4$ über das Sauerstoff- bzw. Schwefelatom erfolgt;

$R^1$-$R^4$

eine $C_1$-$C_6$-Alkylgruppe, die einen $C_3$-$C_8$-Cycloalkylrest tragen kann, eine partiell oder vollständig halogenierte $C_1$-$C_6$-Alkyl-gruppe, eine $C_3$-$C_8$-Cycloalkylgruppe, die noch bis zu 3 $C_1$-$C_6$-Alkylgruppen tragen kann, eine $C_1$-$C_4$-Alkoxy-$C_1$-$C_4$-alkylgruppe, eine $C_1$-$C_4$-Alkylthio-$C_1$-$C_4$-alkylgruppe, eine $C_2$-$C_6$-Alkenylgruppe, eine $C_2$-$C_6$-Alkinylgruppe, die Phenyl- oder Naphthylgruppe, die beide noch 1 oder 2 der folgenden Reste tragen können: Nitro, Cyano, Halogen, $C_1$-$C_4$-Alkyl, partiell oder vollständig halogeniertes $C_1$-$C_4$-Alkyl, $C_1$-$C_4$-Alkoxy, $C_1$-$C_4$-Alkoxyimino, Phenyl oder Phenoxy mit gewünschtenfalls jeweils 1 bis 5 Halogenatomen am Aromat und wobei die Phenyl- oder Naphthylgruppe noch so viele Halogenatome, $C_1$-$C_4$-Alkylreste, partiell oder vollständig halogenierte $C_1$-$C_4$-Alkylreste, und/oder $C_1$-$C_4$-Alkoxyreste tragen kann, daß die Gesamtzahl der Reste 5 beträgt; eine 5- oder 6-gliedrige Heteroarylgruppe mit einem Stickstoff-, Sauerstoff- oder Schwefelatom und gewünschtenfalls bis zu 2 weiteren Stickstoffatomen als Heteroatomen, ausgenommen Heterocyclen mit 3 benachbarten Heteroatomen, wobei der Heteroaromat noch bis zu 3 Halogenatome, $C_1$-$C_4$-Alkylreste, partiell oder vollständig halogenierte $C_1$-$C_4$-Alkylreste oder $C_1$-$C_4$-Alkoxyreste oder bis zu 2 der folgenden Reste tragen kann: Cyano, Nitro, $C_1$-$C_4$-Alkoxyimino, Phenyl oder Phenoxy, die beide am Aromat noch bis zu 5 Halogenatome tragen können;

$R^2$

zusätzlich Wasserstoff, wenn X und Y zusammen einen Rest

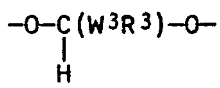 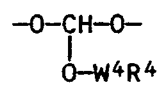 oder 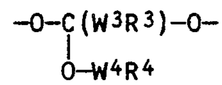

bilden und $W^2$ eine direkte Bindung bedeutet,

ausgenommen cis-1-(2,4-Dichlorphenyl)-2-(pyrid-3-yl)-1,2-epoxypropan, trans-1-(2,4-Dichlorphenyl)-2-(pyrid-3-yl)-1,2-epoxypropan, 1-(2,4-Dichlorphenyl)-2-(pyrid-3-yl)-1,2-epoxybutan und Methansulfonsäure-2,4-dichlor-α-[1-hydroxy-1-(pyrid-3-yl)-ethyl]-benzylester,

sowie die N-Oxide und die pflanzenverträglichen mineralsauren Salze und Metallkomplexe von I.

Außerdem betrifft die Erfindung Verfahren zur Herstellung dieser Verbindungen, ihre Verwendung als

Fungizide und fungizide Mittel, welche diese Verbindungen als wirksame Substanzen enthalten.

Aus Tetrahedron 24, 1959 (1968) sind cis- und trans-1-Phenyl-2-(pyrid-3-yl)-oxiran bekannt, für die jedoch keine biologische Wirkung angegeben wird. Des weiteren sind aus dieser Literaturstelle Pyridylethandiole der Formel II bekannt, z.B. das 1-Phenyl-2-(pyrid-3-yl)ethan-1,2-diol

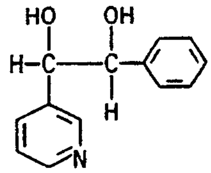

Ferner sind aus der EP-A 74 018 fungizid wirksame 3-substituierte Pyridine und Pyridin-N-Oxide vom Typ der Verbindungen I bekannt, bei denen X eine Hydroxylgruppe, Y Wasserstoff, Halogen, Alkyl, Methylthio oder Methylsulfonyl oder X und Y zusammen Sauerstoff, $W^1$ und $W^2$ eine direkte Bindung, $R^1$ 2,4-Dichlorphenyl und $R^2$ Alkyl bedeuten.

Die fungiziden Wirkungen dieser Verbindungen können jedoch, besonders bei kleinen Aufwandmengen und -konzentrationen, nur bedingt befriedigen.

Der Erfindung lag daher die Aufgabe zugrunde, neue fungizid wirksame Substanzen zu finden.

Demgemäß wurden die eingangs definierten 3-substituierten Pyridine der Formel I gefunden.

Im einzelnen haben die Variablen in den erfindungsgemäßen Verbindungen I die folgende Bedeutung:

X
- die Hydroxylgruppe und

Y
- einen Rest -O-CHO, $-O-CO-W^3R^3$ oder $-O-SO_2-W^3R^3$ oder

X und Y zusammen
- Sauerstoff
- eine Gruppe

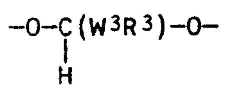 , 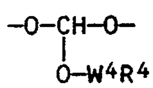 oder 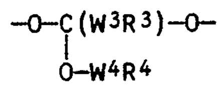 ;

$W^1$, $W^2$, $W^3$, $W^4$
- eine direkte Bindung;
- eine Gruppe $-CH_2-$, $-CH_2-CH_2-$ oder $-CH(CH_3)-$;
- eine Gruppe $-CH_2-O-$ oder $-CH_2-S-$, wobei die Verknüpfung mit den Resten $R^1$ bis $R^4$ über das Sauerstoff- bzw. Schwefelatome erfolgt;

$R^1$, $R^2$, $R^3$, $R^4$
- eine verzweigte oder unverzweigte $C_1$-$C_6$-Alkylgruppe, insbesondere eine $C_1$-$C_4$-Alkylgruppe wie Methyl, Ethyl, n-Propyl, Isopropyl, n-Butyl, 1-Methylprop-1-yl und tert.-Butyl, die einen $C_3$-$C_8$-Cycloalkyl-, insbesondere einen Cyclopropyl-, Cyclopentyl- und Cyclohexylrest tragen kann, beispielsweise Cyclopropylmethyl, 1-Cyclopropylethyl, 2-Cyclopropylethyl, Cyclopentylmethyl, 1-Cyclopentylethyl und 1-Cyclohexylethyl; besonders bevorzugt ist Isopropyl;
- eine verzweigte oder unverzweigte, partiell oder vollständig halogenierte $C_1$-$C_6$-Alkylgruppe, insbesondere eine $C_1$-$C_4$-Alkylgruppe wie Fluormethyl, Chlormethyl, Brommethyl, Difluormethyl, Trifluormethyl, Trichlormethyl, 1-Chlorethyl, Pentafluorethyl, 4-Chlorbut-1-yl und 2-Chlor-1,1,2-trifluorethyl, insbesondere Trifluormethyl;
- eine $C_3$-$C_8$-Cycloalkylgruppe wie Cyclopropyl, Cyclobutyl, Cyclopentyl, Cyclohexyl, Cycloheptyl und Cyclooctyl, insbesondere Cyclopropyl, Cyclopentyl und Cyclohexyl, wobei die Cycloalkylgruppe noch einen bis drei verzweigte oder unverzweigte $C_1$-$C_6$-Alkylsubstituenten, insbesondere $C_1$-$C_4$-Alkylsubstituenten wie Methyl, Ethyl, n-Propyl, Isopropyl, n-Butyl, 1-Methylprop-1-yl und tert.-Butyl, tragen kann; bevorzugt sind 1-Methylcyclopropyl, 2-Methylcyclopropyl, 3-Isopropylcyclohexyl, 4-tert.-Butylcyclohex-1-yl und 2-Isopropyl-5-methylcyclohex-1-yl;
- eine $C_1$-$C_4$-Alkoxy-$C_1$-$C_4$-alkyl- oder $C_1$-$C_4$-Alkylthio-$C_1$-$C_4$-alkylgruppe wie Methoxymethyl, Ethoxymethyl, 1-Methoxyeth-1-yl, 2-Ethoxyethyl, 2-Butoxyethyl, tert.-Butyloxymethyl, Methylthiomethyl, Ethylthiomethyl, tert.-Butylthiomethyl und Methylthioethyl;

- eine $C_2$-$C_6$-Alkenylgruppe wie Vinyl, Allyl, 2-Methylallyl, 3-Methylallyl, Prop-2-en-2-yl, 3,3-Dimethylallyl und 3-Buten-1-yl; besonders bevorzugt ist Vinyl;
- eine $C_2$-$C_6$-Alkinylgruppe, insbesondere eine $C_2$-$C_4$-Alkinylgruppe wie Ethinyl, Propargyl, But-1-in-1-yl und Prop-1-in-1-yl;
- die Phenyl- oder Naphthylgruppe, wobei diese Gruppen noch insgesamt einen bis fünf Reste tragen können, davon insbesondere
  - eine oder zwei Nitrogruppen,
  - eine oder zwei Cyanogruppen,
  - eine oder zwei $C_1$-$C_4$-Alkoxyiminogruppen, insbesondere Methoxyimino und Ethoxyimino,
  - eine oder zwei Phenyl- und/oder Phenoxygruppen, die beide noch bis zu 5 Halogenatome wie Fluor, Chlor, Brom und Jod, bevorzugt Fluor und Chlor, tragen können;
  - bis zu 5 Halogenatome wie Fluor, Chlor, Brom und Jod, bevorzugt Fluor, Chlor und Brom;
  - bis zu 5 $C_1$-$C_4$-Alkylgruppen wie Methyl, Ethyl, n-Propyl, Isopropyl, n-Butyl, 1-Methylprop-1-yl und tert.-Butyl;
  - bis zu 5 partiell oder vollständig halogenierte $C_1$-$C_4$-Alkylgruppen wie Fluormethyl, Chlormethyl, Brommethyl, Difluormethyl, Trifluormethyl, Trichlormethyl, 1-Chlorethyl, Pentafluorethyl, 4-Chlorbut-1-yl und 2-Chlor-1,1,2-trifluorethyl, bevorzugt Trifluormethyl,
  - bis zu 5 $C_1$-$C_4$-Alkoxygruppen wie Methoxy, Ethoxy, n-Propoxy, Isopropoxy, n-Butoxy und tert.-Butoxy;

  besonders bevorzugt sind Phenyl, 2-Nitrophenyl, 4-Nitrophenyl, 2-Cyanophenyl, 4-Cyanophenyl, 2-Cyano-4-chlorphenyl, Biphenyl, 4-Phenoxyphenyl, 4-(4'-Chlorphenoxyphenyl, $C_1$-$C_4$-Alkoxyiminophenyl wie 4-Methoxyiminophenyl und 4-Ethoxyiminophenyl, Halogenphenyl wie 2-Fluorphenyl, 4-Fluorphenyl, 2-Chlorphenyl, 4-Chlorphenyl, 2-Bromphenyl, 4-Bromphenyl, 2-chlor-4-nitrophenyl, 2-Chlor-4-cyanophenyl, 2-Chlor-4-methylphenyl und 2-Brom-4-Methylphenyl, Dihalogenphenyl wie 2,3-Dichlorphenyl, 2,4-Dichlorphenyl, 2,6-Dichlorphenyl, 2-Chlor-3-fluorphenyl, 2-Chlor-4-fluorphenyl, 2-Brom-4-fluorphenyl und 2-Brom-4-chlorphenyl, $C_1$-$C_4$-Alkylphenyl wie 2-Methylphenyl, 4-Methylphenyl und 4-tert.-Butylphenyl, $C_1$-$C_4$-Halogenalkylphenyl wie 2-Trifluormethylphenyl und 4-Trifluormethylphenyl sowie $C_1$-$C_4$-Alkoxyphenyl wie 2-Methoxyphenyl; 4-Methoxyphenyl und 4-tert.-Butoxyphenyl; ganz besonders bevorzugt sind 4-Fluorbenzyl und 2,4-Dichlorbenzyl;
- eine 5- oder 6-gliedrige Heteroarylgruppe mit einem Stickstoff-, Sauerstoff- oder Schwefelatom und gewünschtenfalls zusätzlich 1 oder 2 Stickstoffatomen als Heteroatomen, ausgenommen Heterocyclen mit 3 benachbarten Heteroatomen im Cyclus, beispielsweise Pyrrol-2-yl, Thien-2-yl, Furan-2-yl, Isoxazol-5-yl, Pyrazolyl, 1,3,4-Triazol-2-yl, Pyrid-2-yl, Pyrid-3-yl, Pyrid-4-yl und Pyrimidyl,

  wobei diese Gruppen an den C-Atomen noch bis zu 3-Halogenatome wie vorstehend genannt, insbesondere Fluor, Chlor und Brom, $C_1$-$C_4$-Alkylgruppen wie vorstehend genannt, insbesondere Methyl, Isopropyl und tert.-Butyl und/oder $C_1$-$C_4$-Alkoxygruppen wie vorstehend genannt, insbesondere Methoxy und Ethoxy, oder bis zu 2 der folgenden Reste tragen können: Nitro, Cyano, $C_1$-$C_4$-Alkoxyimino, insbesondere Methoxyimino und Ethoxyimino sowie Phenyl oder Phenoxy, die beide noch bis zu 5 Halogenatome wie Fluor, Chlor, Brom und Jod, insbesondere Fluor und Chlor, tragen können, beispielsweise 2-Chlorthien-3-yl, 3-Bromthien-2-yl, 3-Isopropylisoxazol-5-yl und 3-Phenylisoxazol-5-yl;

$R^2$

zusätzlich Wasserstoff, wenn X und Y zusammen einen Rest

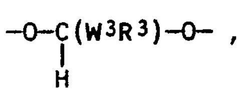 , 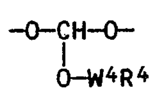 oder 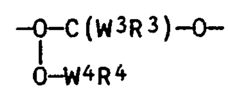

bilden und $W^2$ eine direkte Bindung bedeutet.

Besonders bevorzugte Substituenten I sind den Tabellen der Beispiele zu entnehmen.

Ganz besonders bevorzugt sind 1-Acetoxy-1-(2,4-dichlorphenyl)-2-(pyrid-3-yl)-3-methyl-butan-2-ol, 1-(2,4-Dichlorphenyl)-1-methylsulfonyloxy-2-(pyrid-3-yl)-hexan-2-ol, 1-Isopropyl-1-(pyrid-3-yl)-2-(2,4-dichlorphenyl)-oxiran, 2-Methoxy-2-methyl-4-(pyrid-3-yl)-4-isopropyl-5-(2,4-dichlorphenyl)-1,3-dioxolan, 2-Methoxy-2-methyl-4-(pyrid-3-yl)-4-vinyl-5-(2,4-dichlorphenyl)-1,3-dioxolan, 2-Methoxy-2-methyl-4-(4-fluorphenyl)-4-(pyrid-3-yl)-5-(2,4-dichlorphenyl)-1,3-dioxolan, 2-(4-Methylphenyl)-4-(pyrid-3-yl)-5-(2-bromphenyl)-1,3-dioxolan, 2-Phenyl-4-(pyrid-3-yl)-5-(2,4-dichlorphenyl)-1,3-dioxolan, 2-Phenyl-4-(pyrid-3-yl)-5-(2-chlorphenyl)-1,3-dioxolan und 2-(4-Methylphenyl)-4-(pyrid-3-yl)-5-(2-chlorphenyl)-1,3-dioxolan.

Die 3-substituierten Pyridine sind auf verschiedene Weise erhältich, und zwar vorzugsweise nach

folgenden Methoden:

a) Herstellung von Verbindungen I, bei denen X eine Hydroxylgruppe und Y einen Rest -O-CHO, -O-CO-$W^3R^3$ oder -O-$SO_2$-$W^3R^3$ bedeuten:

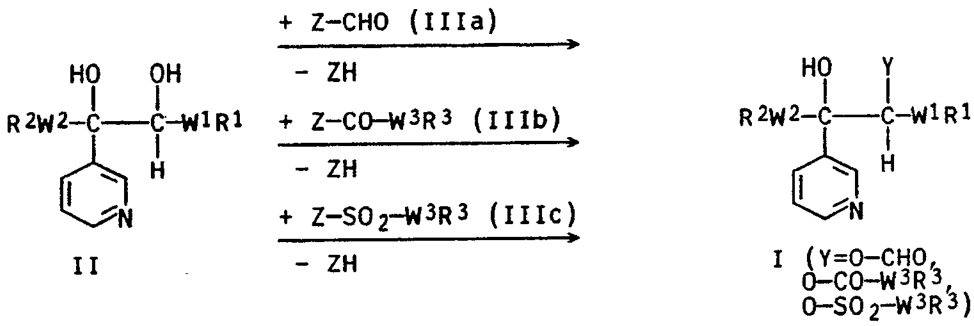


II

I (Y=O-CHO, O-CO-$W^3R^3$, O-$SO_2$-$W^3R^3$)

Z bedeutet Halogen, insbesondere Chlor und Brom, Acyloxy oder Sulfonyloxy.

Die Reaktion wird zweckmäßigerweise in einem Lösungs- oder Verdünnungsmittel bei Normaldruck durchgeführt, besonders bevorzugt unter Zusatz einer organischen oder anorganischen Base. Geringerer oder höherer Druck ist möglich, bringt im allgemeinen aber keine Vorteile.

Als Lösungs- bzw. Verdünnungsmittel können aliphatische Kohlenwasserstoffe wie n-Pentan, n-Hexan und Cyclohexan, aromatische Kohlenwasserstoffe wie Toluol und o-, m-, p-Xylol, chlorierte Kohlenwasserstoffe wie Methylenchlorid, Chloroform und Tetrachlorkohlenstoff, Ether wie Diethylether und Tetrahydrofuran sowie Ester wie Essigester verwendet werden.

Als Basen eignen sich beispielsweise Alkalimetallhydroxide, insbesondere Natrium- und Kaliumhydroxid, Alkalimetallcarbonate wie Natrium- und Kaliumcarbonat, Hydrogencarbonate wie Natrium- und Kaliumhydrogencarbonat sowie Amine wie Triethylamin, Pyridin und 4-Dimethylaminopyridin, und zwar für eine vollständige Umsetzung in mindestens stöchiometrischer Menge, bezogen auf die Menge an II.

Die Verbindungen II können auch zuerst in einem getrennten Verfahrensschritt mittels Base in die entsprechenden Alkoholate überführt werden.

Bedeutet Z Acyloxy oder Sulfonyloxy, so empfiehlt sich die Verwendung von tertiären Aminen wie Triethylamin oder Pyridin als Base, wobei man die Umsetzung besonders bevorzugt lösungsmittelfrei in einem überschuß an Base durchführt.

Durch Zusatz eines Katalysators, bevorzugt 4-Dimethylaminopydridin, kann gegebenenfalls die Reaktionsgeschwindigkeit erhöht werden [vgl. Angew. Chemie 90, 602 (1978)].

Normalerweise setzt man alle Ausgangsverbindungen in etwa stöchiometrischem Verhältnis ein, jedoch kann in manchen Fällen auch ein überschuß der einen oder anderen Komponente, etwa bis zu 10 %, empfehlenswert sein.

Verwendet man eine organische Base gleichzeitig als Lösungsmittel, so liegt sie in einem größeren überschuß vor.

Im allgemeinen liegt die Reaktionstemperatur zwischen 0 °C und 100 °C, insbesondere bei der Rückflußtemperatur des jeweiligen Lösungsmittels.

Die Pyridylethandiole II sind z.B. aus folgenden Druckschriften bekannt: JP-A 48/5593, Tetrahedron 24, 1959 (1968), J. Org. Chem. 52, 957 (1987), Helv. Chem. Acta 68, 600 (1985), Chem. Heterocycl. Compounds 10, 210 (1974), Acta Pharm. Succ. 9 (4), 289 (1972) und EP-A 209 854. Weitere Derivate II können nach den in vorstehenden Druckschriften genannten Methoden oder nach den in der Deutschen Patentanmeldung P 3943277 beschriebenen Verfahren hergestellt werden.

Die Ausgangsverbindungen IIIa bis IIIc sind bekannt oder können nach bekannten Verfahren hergestellt werden.

b) Herstellung von Verbindungen I, bei denen X und Y zusammen ein Sauerstoffatom bedeuten, durch Umsetzung von 3-substituierten Pyridinen Ia mit einer Base:

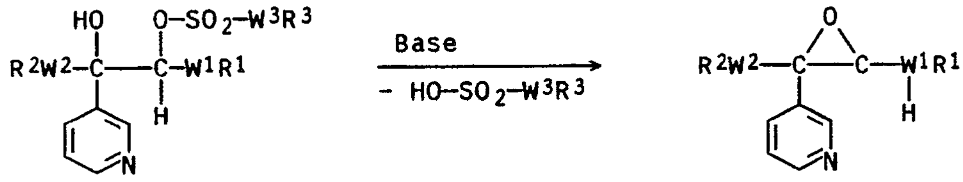


Ia

(I; X=OH, Y=O-SO$_2$-W$^3$R$^3$)

I (X+Y=O)

Die Reaktion wird zweckmäßigerweise in einem inerten Lösungs- oder Verdünnungsmittel bei Normaldruck durchgeführt, wobei die Reaktionstemperatur im allgemeinen zwischen 20 °C und der Rückflußtemperatur des jeweiligen Lösungsmittels liegt.

Die Reaktion erfolgt normalerweise in einem inerten Lösungs- oder Verdünnungsmittel nach an sich bekannten Verfahren, wobei als Basen beispielsweise Alkalimetallhydroxide wie Kaliumhydroxid [vgl. Furst, Helv. 32, 1454 (1954)], Alkalimetallalkoxide wie Natriummethanolat [vgl. Reist, J. Org. Chem. 30, 3401 (1965)], Tetraalkylammoniumhydroxide wie Triethylammoniumhydroxid [vgl. Marshall, THL 27, 5197 (1986)] und tert. Amine wie Triethylamin [vgl. Ogate, J. Med. Chem. 30, 1054 (1987)] geeignet sind.

Bezüglich der Lösungsmittel, des Druckes, der Temperatur und der Mengenverhältnisse gelten die Angaben für Methode (a).

In einer ganz besondes bevorzugten Ausführungsform stellt man die 3-substituierten Pyridine I (X+Y=Sauerstoff) in einer 1-stufigen Synthese direkt aus den Pyridylethandiolen II her, indem man diese zusammen mit den Verbindungen IIIa, IIIb oder IIIc in Gegenwart einer überschüssigen Menge an Base umsetzt. Als Base ist Triethylamin besonders geeignet. Zweckmäßigerweise arbeitet man in einem inerten Verdünnungsmittel, insbesondere in Methylenchlorid.

Die Mengenverhältnisse sind nicht kritisch. Normalerweise werden die Edukte II und IIIa, IIIb oder IIIc in stöchiometrischem Verhältnis eingesetzt, jedoch kann auch ein überschuß dir Verbindungen IIIa, IIIb oder IIIc, etwa bis zu 50 %, vorteilhaft sein.

Im allgemeinen arbeitet man unter Normaldruck oder unter dem Eigendruck des jeweiligen Lösungsmittels, wobei sich eine Reaktionstemperatur zwischen 20 °C und dem siedepunkt des Lösungsmittels empfiehlt.

c) Herstellung von Verbindungen I, bei denen X und Y zusammen eine Gruppe

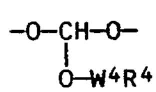

oder

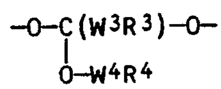

bedeuten:

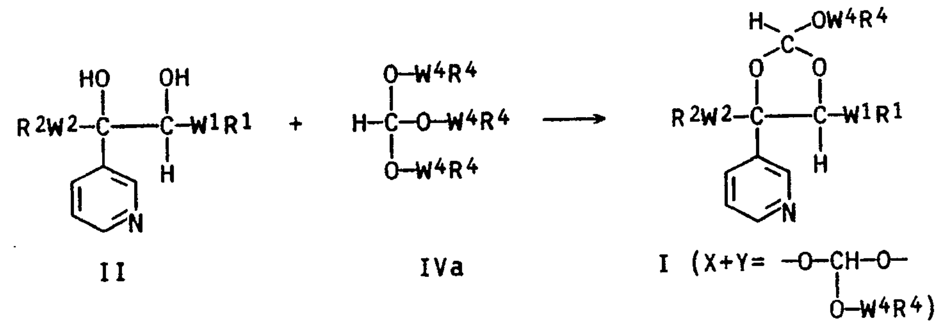

II IVa I

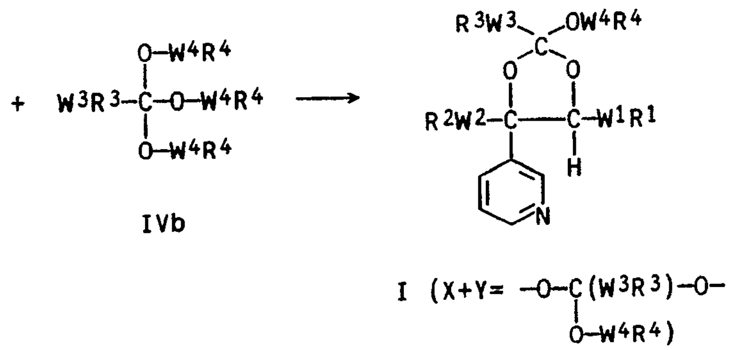

IVb I

Die Umsetzung erfolgt zweckmäßig in einem inerten Lösungs- oder Verdünnungsmittel, beispielsweise in einem Kohlenwasserstoff wie Toluol, o-, m-, p-Xylol oder in einem Halogenkohlenwasserstoff wie Methylenchlorid, Chloroform und 1,2-Dichlorethan oder in einem überschuß des Orthoesters IVa bzw. IVb.

Besonders vorteilhaft ist es, die Reaktion in Gegenwart einer starken Säure wie Salzsäure, Schwefelsäure und para-Toluolsulfonsäure durchzuführen, wobei die Menge an Säure nicht kritisch ist.

Normalerweise verwendet man einen überschuß des Orthoesters IVa oder IVb bis 30 mol-% bezogen auf die Menge an Pyridinethandiol II, jedoch kann in manchen Fällen auch das Arbeiten ohne Lösungsmittel in einem größeren überschuß an Orthoester empfehlenzwert sein.

Im allgemeinen arbeitet man bei einer Reaktionstemperatur zwischen 20 und 120 °C oder bevorzugt bei der Siedetemperatur des verwendeten Lösungsmittels.

Zweckmäßigerweise arbeitet man bei Atmosphärendruck oder unter dem Eigendruck des jeweiligen Lösungsmittels. In einer besonders bevorzugten Verfahrensvariante setzt man die Pyridylethandiole II mit Trimethyl- oder Triethylorthoessigsäureester zu Verbindungen I um, bei denen W eine direkte Bindung und $R^4$ Methyl oder Ethyl bedeuten. Diese Produkte können dann in einem weiteren Verfahrensschritt durch Umsetzung mit Alkoholen $R^4W^4$-OH in Gegenwart einer Säure in weitere 3-substituierten Pyridine I übergeführt werden ([vgl. De Wolfe, Synthesis, 165 (1974)]:

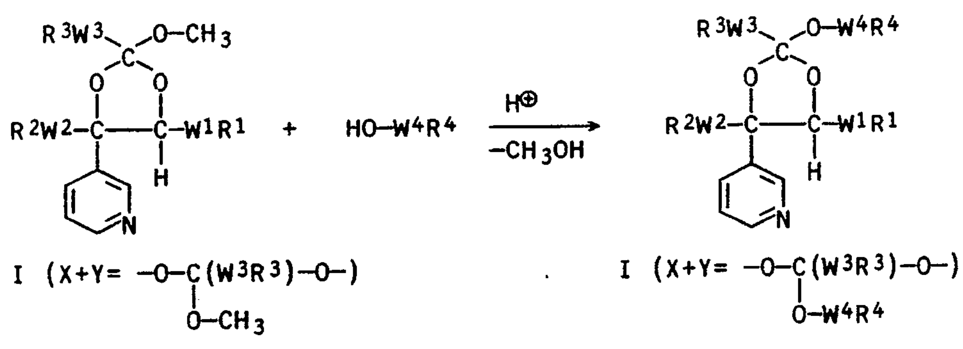


I I

Bevorzugt führt man diese Reaktion lösungsmittelfrei in einem überschuß des jeweiligen Alkohols HO-$W^4R^4$ durch, wobei das Methanol zweckmäßigerweise in dem Maße, wie es entsteht, aus dem

Reaktionsgemisch abdestilliert wird.

d) Herstellung von Verbindungen I, bei denen X und Y zusammen eine Gruppe

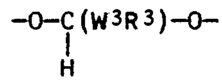 bedeuten:

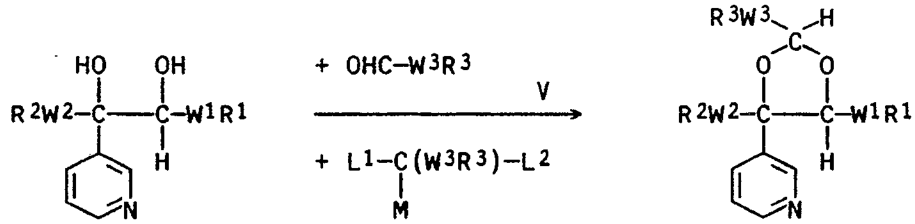


II VI I (X+Y = 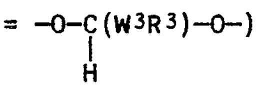

$L^1$, $L^2$ = Halogen, bevorzugt Chlor oder Brom, oder $C_1$-$C_4$-Alkoxy, bevorzugt Methoxy oder Ethoxy.

Acetalisierungsreaktionen mit Verbindungen der Formel V sind allgemein bekannte Reaktionen der organischen Chemie. Sie werden gewöhnlich zur Reaktionsbeschleunigung in Gegenwart einer starken Säure (Organikum, S. 490, 15. Aufl., 1977) oder einer Lewis-Säure (R. Masuda, Tetrahedron Letters 26, S. 4767, 1977) durchgeführt. In einer bevorzugten Ausführungsvariante werden z.B. beide Reaktionspartner zusammen mit einem Aceotrop-bildenden Lösungsmittel in Gegenwart einer starken Säure wie p-Toluolsulfonsäure unter Rückfluß erhitzt. Als Lösungsmittel kommen z.B. Chloroform, Tetrachlorkohlenstoff, Chlorbenzol, aromatische Kohlenwasserstoffe wie Benzol, Toluol, Xylol oder auch Gemische derselben in Betracht.

Reaktionen mit Verbindungen der Formel VI entsprechen, wenn $L^1$ und $L^2$ Alkoxy wie Methoxy und Ethoxy bedeuten, einer Umacetalisierungsreaktion. Diese werden gewöhnlich ebenfalls unter Zusatz einer starken Säure durchgeführt (J. March, Adv. Org. Chem., S. 345, 3. Aufl., 1985). Eine weitere an sich bekannte Methode zur Darstellung cyclischer Acetale besteht darin, daß man geminale Dihalogenide, insbesondere Chloride und/oder Bromide, in Gegenwart von Stickstoffbasen wie Pyridin zur Cyclisierung einsetzt (P.J. Garegg et al., Acta Chem. Scand. 26, S. 518ff und S. 3895ff, 1972).

Besonders bevorzugt ist die Umsetzung von Pyridylethandiolen II mit einem überschuß eines Dimethylacetals VI ($L^1$,$L^2$=$OCH_3$) oder eines Diethylacetals IV ($L^1$,$L^2$=$OC_2H_5$) in Gegenwart einer starken Säure wie Schwefelsäure und p-Toluolsulfonsäure, ggf. unter Zusatz eines Lösungsmittels, wobei die Temperatur bis zum Siedepunkt der Reaktionslösung ansteigen kann. Als Lösungsmittel kommen z.B. Chloroform, Tetrachlorkohlenstoff, Chlorbenzol, aromatische Kohlenwasserstoffe wie Benzol, Toluol, Xylol oder auch Gemische derselben in Frage.

Pyridylethandiole der Formel II, in denen $R^2$ Wasserstoff und $W^2$ eine direkte Bindung bedeuten, erhält man durch Reduktion von Acyloinen der Formel VIIa oder VIIb

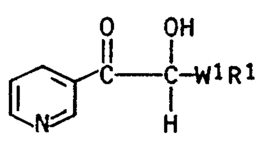 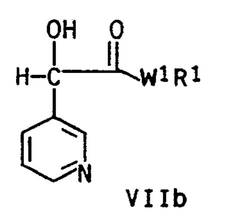

VIIa VIIb

in denen $R^1$ und $W^1$ die oben genannten Bedeutungen haben. Als Reduktionsmittel kommen Metallhydride oder Wasserstoff in Gegenwart eines Katalysators in Frage (vgl. z.B. M. Hudlicky, Reductions in Org. Chem. S. 119f, 1984). Geeignete Metallhydride sind z.B. Diisobutylaluminiumhydrid, Lithium-, Natrium-, Kalium- und Zinkborhydrid oder -cyanoborhydrid, aber auch Lithiumaluminiumhydride der allgemeinen Formel $LiAl(H)_m(OR)_n$ mit m=1 bis 4 und n=4-m, wobei R für Alkylreste wie Methyl, Ethyl, Propyl, iso-Propyl, n-Butyl, iso-Butyl, tert.-Butyl und Cyclohexyl steht. Ganz besonders bevorzugt ist Natriumborhydrid.

Die Reaktion wird im allgemeinen zwischen (-50)°C und 80°C, besonders bevorzugt bei (-20)°C bis

50° C, ganz besonders bei (-10)° C bis 30° C durchgeführt. Als Lösungsmittel kommen Alkohole wie Methanol, Ethanol, iso-Propanol, tert.-Butanol, Ether wie Diethylether, Methyl-tert.-butylether, Tetrahydrofuran und Dimethoxyethan in Betracht.

Pyridylethandiole der Formel II, in denen $R^1$, $R^2$, $W^1$ und $W^2$ die oben genannten Bedeutungen haben mit der Ausnahme, daß $R^2$ nicht Wasserstoff ist, erhält man dadurch, daß man eine Organometallverbindung der Formel VIII an ein Acyloin der Formel VIIa addiert.

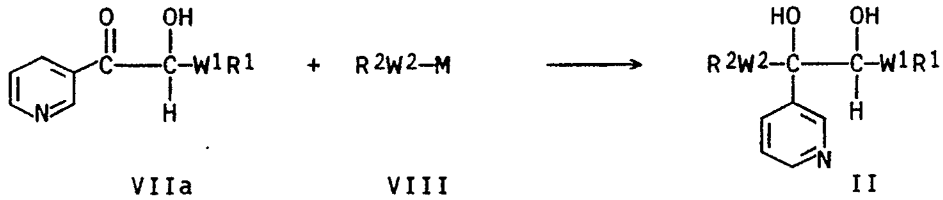

VIIa VIII II

In Formel VIII haben $R^2$ und $W^2$ die oben genannten Bedeutungen mit der Ausnahme, daß $R^2$ nicht Wasserstoff ist. M steht für Lithium oder die Reste MgCl oder MgBr.

Zweckmäßig wird die Reaktion so durchgeführt, daß man 2 bis 4 Äquivalente der Organometallverbindung in einem inerten Lösungsmittel, vorzugsweise Diethylether, Methyl-tert.-butylether, Tetrahydrofuran oder einem Gemisch derselben, vorlegt und ein Acyloin der Formel VIIa bei (-30)° C bis 50° C zudosiert. Es ist auch möglich in dieser Reaktion anstelle der Acyloine VIIa Derivate der Formel VIIa'

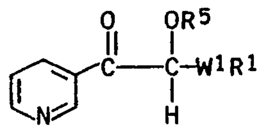

VIIa'

einzusetzen, in der $W^1$ und $R^1$ die oben genannten Bedeutungen haben und $R^5$ für eine Schutzgruppe der OH-Funktion (T.W. Greene, Protective Groups in Org. Synth., S. 10-113, 1981) steht, die nach der Reaktion wieder abgespalten werden kann. Bevorzugt sind hierbei Ester wie Acetat und Benzoat aber auch Ether wie Methoxymethyl- und Trimethylsilylether zu nennen.

Pyridylethandiole der Formel II, in denen $R^1$ für Wasserstoff, $W^1$ für eine direkte Bindung stehen und $R^2$ und $W^2$ die oben genannten Bedeutungen haben, lassen sich durch Umsetzung von 3-Pyridylketonen der Formel IX mit Dimethylsulfonium- oder Diethylsulfoxoniummethylid herstellen:

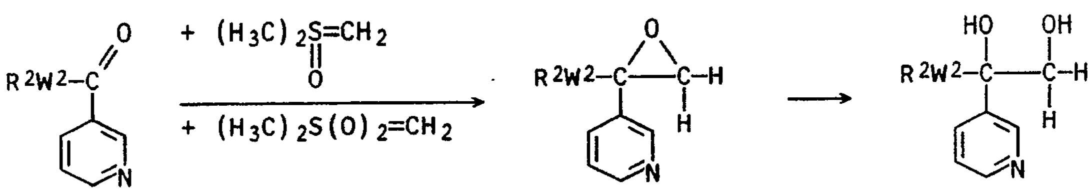


IX X II

3-Pyridylketone der Formel IX sind allgemein bekannte Verbindungen, die sich durch eine Vielzahl von bekannten Methoden herstellen lassen. Eine bevorzugte Methode besteht z.B. in der Alkylierung von alpha-Morpholino-3-pyridylacetonitril und nachfolgender Freisetzung der Ketonfunktion (E. Leete et al, JOC 37, S. 4465, 1972) oder in der Addition von 3-Pyridyllithium (Wibaut et al, Rec. Trav. chim. 77, S. 1057, 1958) an entsprechend substituierte Nitrile mit anschließender saurer Aufarbeitung. Die Bildung von Oxiranen X durch Umsetzung von 3-Pyridylketonen mit Dimethylsulfonium- oder Dimethylsulfoxoniummethylid ist bekannt (M. Sainsbury et al, JCS Perk. Trans. 1, S. 587ff, 1982). Die öffnung der Epoxide X zu den Pyridylethandiolen II erreicht man durch Zusatz von Säuren oder Basen (J. March, Adv. Org. Chem. 3rd Ed., S. 332, 1985).

Pyridylethandiole der allgemeinen Formel II'

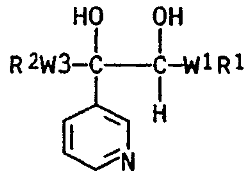

II'

in welcher die Reste $R^1$, $R^2$, $W^1$ und $W^2$ die oben genannten Bedeutungen haben, mit der Maßgabe, daß, wenn $R^2W^2$ für Wasserstoff steht, $R^1W^1$ nicht Wasserstoff, Vinyl, unsubstituiertes Phenyl oder 3-Pyridyl bedeutet, und der Maßgabe, wenn $R^2W^2$ für Methyl steht, $R^1W^1$ nicht 2,4-Dichlorphenyl bedeutet, und der Maßgabe, wenn $R^1W^1$ für unsubstituiertes Phenyl steht, $R^2W^2$ nicht unsubstituiertes Phenyl oder 4-Methoxyphenyl bedeuten, und der Maßgabe, daß, wenn $R^1$ für Wasserstoff oder $C_1$-$C_5$-Alkyl, $R^2$ für gegebenenfalls substituiertes Phenyl und $W^1$ für eine direkte Bindung stehen, $W^2$ nicht $-CH_2-$ oder $-CH(CH_3)$-bedeutet, sind neu.

Auch die Acyloine der Formel VIIa und VIIb sind neu. Acyloine der Formel VIIa lassen sich z.B. durch Addition von 3-Pyridylmagnesiumbromid oder -chlorid an O-Trimethylsilylcyanhydrine (J.K. Rasmussen et al, THL 24, S. 4075ff, 1983) herstellen. Bevorzugt wird aber die Acyloinkondensation von 3-Pyridylaldehyd mit Aldehyden der Formel XI

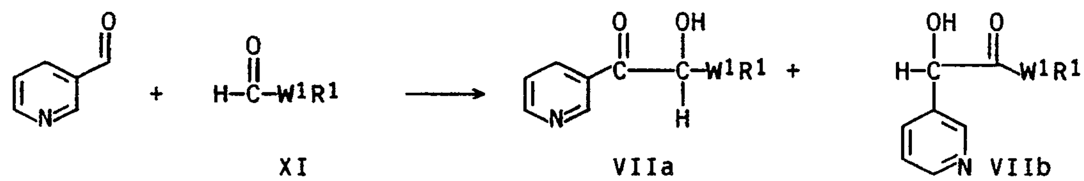

XI VIIa VIIb

wobei $R^1$ und $W^1$ die oben genannten Bedeutungen haben. Man kann die Reaktion durch Cyanidionen (P. Bergmann, H. Paul, Z. Chem., S. 339ff, 1966) oder auch durch Thiazoliumsalze (H. Stetter et al., Synthesis, S. 733, 1976) katalysieren. Thiazoliumsalz-katalysierte Acyloinkondensationen mit 3-Pyridylaldehyd waren bisher unbekannt. Es ist deshalb als sehr überraschend zu bezeichnen, daß in vielen Fällen sehr hohe Ausbeuten an den gemischten Acyloinen VIIa und VIIb erzielt werden, die symmetrischen Acyloine der Formeln XIIa und XIIb nicht oder allenfalls als Nebenprodukte auftreten:

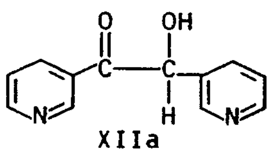

XIIa

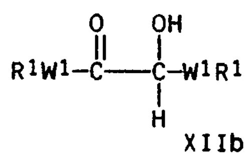

XIIb

Oft erhält man auch von den beiden Acyloinen VIIa und VIIb das Acyloin VIIa im überschuß.

Die N-Oxide der 3-substituierten Pyridine I können nach üblichen Methoden aus den Verbindungen I, z.B. durch deren Umsetzung mit einer organischen Persäure wie Metachlorperbenzoesäure, hergestellt werden.

Als Säureadditionssalze eignen sich die Salze von solchen Säuren, welche die fungizide Wirkung von I nicht beeinträchtigen, also z.B. die Salze der Chlorwasserstoffsäure, Bromwasserstoffsäure, Schwefelsäure, Salpetersäure, Phosphorsäure, Borsäure, Ameisensäure, Essigsäure, Propionsäure, Laurylsäure, Palmitinsäure, Stearinsäure, Oxalsäure, Äpfelsäure, Malonsäure, Benzoesäure, Methansulfonsäure, Benzolsulfonsäure, Toluolsulfonsäure, Dodecylbenzolsulfonsäure sowie der Saccharinsäure.

Als Metallkomplexe kommen die Komplexe des Kupfers, Zinks, Zinns, Mangans, Eisens, Kobalts oder Nickels in Betracht. Vorzugsweise stellt man die Komplexe aus den freien Basen I und mineralsauren Salzen, beispielsweise den Chloriden oder Sulfaten, der Metalle her.

Die erfindungsgemäßen Verbindungen I können in mehreren, mindestens jedoch in 2 isomeren Formen auftreten. Bei den meisten Herstellungsverfahren werden Gemische der möglichen Isomeren erhalten, im allgemeinen Racemate oder Diastereomerengemische, die sich gewünschtenfalls aber nach den hierfür üblichen Methoden, z.B. durch Chromatographie an einem optisch aktiven Adsorptionsmittel, in die reinen

Isomeren auftrennen lassen.

Die 3-substituierten Pyridine I eignen sich, sowohl als Racemate oder Diastereomerengemische als auch in Form der reinen Isomeren, als Fungizide.

Die 3-substituierten Pyridine I von Cyclopropancarbonsäuren I zeichnen sich durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden.

Besondere Bedeutung haben sie für die Bekämpfung einer Vielzahl von Pilzen an verschiedenen Kulturpflanzen wie Weizen, Roggen, Gerste, Hafer, Reis, Mais, Gras, Baumwolle, Soja, Kaffee, Zuckerrohr, Wein, Obst- und Zierpflanzen und Gemüsepflanzen wie Gurken, Bohnen und Kürbisgewächsen, sowie an den Samen dieser Pflanzen.

Speziell eignen sie sich zur Bekämpfung folgender Pflanzenkrankheiten: Erysiphe graminis (echter Mehltau) in Getreide,
Erysiphe cichoracearum und Sphaerotheca fuliginea an Kürbisgewächsen,
Podosphaera leucotricha an Äpfeln,
Uncinula necator an Reben,
Puccinia-Arten an Getreide,
Rhizoctonia-Arten an Baumwolle und Rasen,
Ustilago-Arten an Getreide und zuckerrohr,
Venturia inaequalis (Schorf) an Äpfeln,
Helminthosporium-Arten an Getreide,
Septoria nodorum an Weizen,
Botrytis cinerea (Grauschimmel) an Erdbeeren, Reben,
Cercospora arachidicola an Erdnüssen,
Pseudocercosporella herpotrichoides an Weizen, Gerste,
Pyricularia oryzae an Reis,
Phytophthora infestans an Kartoffeln und Tomaten,
Fusarium- und Verticillium-Arten an verschiedenen Pflanzen,
Plasmopara viticola an Reben,
Alternaria-Arten an Gemüse und Obst.

Die Verbindungen werden angewendet, indem man die Pilze oder die vor Pilzbefall zu schützenden Pflanzen, Saatgüter, Materialien oder den Erdboden mit einer fungizid wirksamen Menge der Wirkstoffe behandelt. Die Anwendung erfolgt vor oder nach der Infektion der Materialien, Pflanzen oder Samen durch die Pilze.

Abgesehen von ihrer Wirkung gegen Pilze können die 3-substituierten Pyridine, auch als Herbizide eingesetzt werden. Besonders bevorzugt werden hierzu Verbindungen I, bei denen X und Y zusammen eine Gruppe

$$-O-\underset{\displaystyle O-W^4R^4}{\underset{|}{C}}(W^3R^3)-O- \quad \text{bilden.}$$

Die Wirkstoffe können in die üblichen Formulierungen übergeführt werden, wie Lösungen, Emulsionen, Suspensionen, Stäube, Pulver, Pasten und Granulate. Die Anwendungsformen richten sich nach den Verwendungszwecken; sie sollen in jedem Fall eine feine und gleichmäßige Verteilung der 3-substituierten Pyridine gewährleisten. Die Formulierungen werden in bekannter Weise hergestellt, z.B. durch Verstrecken des Wirkstoffs mit Lösungsmitteln und/oder Trägerstoffen, gewünschtenfalls unter Verwendung von Emulgiermitteln und Dispergiermitteln, wobei im Falle von Wasser als Verdünnungsmittel auch andere organische Lösungsmittel als Hilfslösungsmittel verwendet werden können.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Benzol, Toluol, Xylol, Paraffin, Tetrahydronaphthalin, alkylierte Naphthaline oder deren Derivate, Methanol, Ethanol, Propanol, Butanol, Chloroform, Tetrachlorkohlenstoff, Cyclohexanol, Cyclohexanon, Chlorbenzol, Isophoron, stark polare Lösungsmittel, z.B. Dimethylformamid, Dimethylsulfoxid, N-Methylpyrrolidon, Wasser, in Betracht.

Wäßrige Anwendungsformen können aus Emulsionskonzentraten, Pasten oder netzbaren Pulvern

(Spritzpulver, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermitttel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Dibutylnaphthalinsulfonsäure, Alkylarylsulfonate, Alkylsulfate, Alkylsulfonate, Fettalkoholsulfate und Fettsäuren sowie deren Alkali- und Erdalkalisalze, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphtalinsulfonsäure mit Phenol und Formaldehyd, Polyoxyethylenoctylphenolether, ethoxyliertes Isooctylphenol, Octylphenol, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Lignin-Sulfitablaugen und Methylcellulose in Betracht.

Pulver-, Streu- und Stäubemittel können durch Mischen oder gemeinsames Vermahlen der wirksamen Substanzen mit einem festen Trägerstoff hergestellt werden.

Die Formulierungen enthalten im allgemeinen zwischen 0,01 und 95 Gew.%, vorzugsweise zwischen 0,1 und 90 Gew.% des Wirkstoffs. Die Wirkstoffe werden dabei in einer Reinheit von 90 % bis 100 %, vorzugsweise 95 % bis 100 % (nach NMR-Spektrum) eingesetzt.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden, wie Silicagel, Kieselsäuren, Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Beispiele für solche Zubereitungen sind:

I. eine Lösung aus 90 Gew.-Teilen der Verbindung Nr. 1.01 und 10 Gew.-Teilen N-Methyl-α-pyrrolidon, die zur Anwendung in Form kleinster Tropfen geeignet ist;

II. eine Mischung aus 20 Gew.-Teilen der Verbindung Nr. 2.01, 80 Gew.Teilen Xylol, 10 Gew.-Teilen des Anlagerungsproduktes von 8 bis 10 Mol Ethylenoxid an 1 Mol Ölsäure-N-monoethanolamid, 5 Gew.Teilen Calciumsalz der Dodecylbenzolsulfonsäure, 5 Gew.-Teilen des Anlagerungsproduktes und 40 Mol Ethylenoxid an 1 Mol Ricinusöl; durch feines Verteilen der Lösung in Wasser erhält man eine Dispersion.

III. eine wäßrige Dispersion aus 20 Gew.-Teilen der Verbindung Nr. 2.02, 40 Gew.-Teilen Cyclohexanon, 30 Gew.-Teilen Isobutanol, 20 Gew.-Teilen des Anlagerungsproduktes von 40 mol Ethylenoxid an 1 mol Ricinusöl;

IV. eine wäßrige Dispersion aus 20 Gew.-Teilen der Verbindung Nr. 2.04, 25 Gew.-Teilen Cyclohexanol, 65 Gew.-Teilen einer Mineralölfraktion vom Siedepunkt 210 bis 280 °C und 10 Gew.-Teilen des Anlagerungsproduktes von 40 mol Ethylenoxid an 1 mol Ricinusöl;

V. eine in einer Hammermühle vermahlene Mischung aus 80 Gew.-Teilen der Verbindung Nr. 3.01, 3 Gew.-Teilen des Natriumsalzes der Diisobutylnaphtalin-α-sulfonsäure, 10 Gew.-Teilen des Natriumsalzes einer Ligninsulfonsäure aus einer Sulfitablauge und 7 Gew.-Teilen pulverförmigem Kieselsäuregel; durch feines Verteilen der Mischung in Wasser erhält man eine Spritzbrühe;

VI. eine innige Mischung aus 3 Gew.-Teilen der Verbindung Nr. 3.02 und 97 Gew.-Teilen feinteiligem Kaolin; dieses Stäubemittel enthält 3 Gew.-% Wirkstoff;

VII. eine innige Mischung aus 30 Gew.-Teilen der Verbindung Nr. 3.16, 92 Gew.-Teilen pulverförmigem Kieselsäuregel und 8 Gew.-Teilen Paraffinöl, das auf die Oberfläche dieses Kieselsäuregels gesprüht wurde; diese Aufbereitung gibt dem Wirkstoff eine gute Haftfähigkeit;

VIII. eine stabile wäßrige Dispersion aus 40 Gew.-Teilen der Verbindung Nr. 4.01, 10 Gew.-Teilen des Natriumsalzes eines Phenosulfonsäure-harnstoff-formaldehyd-Kondensates, 2 Gew.-Teilen Kieselgel und 48 Gew.-Teilen Wasser, die weiter verdünnt werden kann;

IX. eine stabile ölige Dispersion aus 20 Gew.-Teilen der Verbindung Nr. 4.02, 2 Gew.-Teilen des Calciumsalzes der Dodecylbenzolsulfonsäure, 8 Gew.-Teilen Fettalkohol-polyglykolether, 20 Gew.-Teilen des Natriumsalzes eines Phenolsulfonsäure-harnstoff-formaldehyd-Kondensates und 68 Gew.-Teilen eines paraffinischen Mineralöls.

Formulierungen mit mehr als 95 Gew.-% Wirkstoff können mit gutem Erfolg im Ultra-Low-Volume-Verfahren (ULV) ausgebracht werden, wobei sogar der Wirkstoff ohne Zusätze verwendet werden kann.

Die Mittel bzw. die daraus hergestellten gebrauchsfertigen Zubereitungen wie Lösungen, Emulsionen, Suspensionen, Pulver, Stäube, Pasten oder Granulate werden in bekannter Weise angewendet, beispielsweise durch Versprühen, Vernebeln, Verstäuben, Verstreuen, Beizen oder Gießen.

Die Aufwandmengen in fungiziden Mitteln liegen je nach Art des gewünschten Effektes zwischen 0,02 und 3 kg Wirkstoff pro ha. Die neuen Verbindungen können auch im Materialschutz (Holzschutz) eingesetzt werden, z.B. gegen Paecilomyces variotii.

Bei der Saatgutbehandlung werden im allgemeinen Wirkstoffmengen von 0,001 bis 50 g, vorzugsweise 0,01 bis 10 g je Kilogramm Saatgut benötigt.

Die Applikation von herbizid wirksamen Mitteln bzw. Wirkstoffen kann im Vorauflauf- oder im Nächauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff betragen je nach Bekämpfungsziel, Jahreszeit, Zielpflanzen und Wachstumsstadium 0,001 bis 5,0, vorzugsweise 0,01 bis 1,0 kg/ha aktive Substanz (a.S.).

In Anbetracht der Vielseitigkeit der Applikationsmethoden können die erfindungsgemäßen Verbindungen bzw. sie enthaltende Mittel noch in einer weiteren Zahl von Kulturplfanzen zur Beseitigung unerwünschter Pflanzen eingesetzt werden. In Betracht kommen beispielsweise folgende Kulturen:

| Botanischer Name | Deutscher Name |
|---|---|
| Allium cepa | Küchenzwiebel |
| Ananas comosus | Ananas |
| Arachis hypogaea | Erdnuß |
| Asparagus officinalis | Spargel |
| Avena sativa | Hafer |
| Beta vulgaris spp. altissima | Zuckerrübe |
| Beta vulgaris spp. rapa | Futterrübe |
| Beta vulgaris spp. esculenta | Rote Rübe |
| Brassica napus var. napus | Raps |
| Brassica napus var. napobrassica | Kohlrübe |
| Brassica napus var. rapa | Weiße Rübe |
| Brassica rapa var. silvestris | Rüben |
| Camellia sinensis | Teestrauch |
| Carthamus tinctorius | Saflor - Färberdistel |
| Carya illinoinensis | Pekannußbaum |
| Citrus limon | Zitrone |
| Citrus maxima | Pampelmuse |
| Citrus reticulata | Mandarine |

| Botanischer Name | Deutscher Name |
|---|---|
| Citrus sinensis | Apfelsine, Orange |
| Coffea arabica (Coffea canephora, Coffea liberica) | Kaffee |
| Cucumis melo | Melone |
| Cucumis sativus | Gurke |
| Cynodon dactylon | Bermudagras |
| Daucus carota | Möhre |
| Elaeis guineensis | Ölpalme |
| Fragaria vesca | Erdbeere |
| Glycine max | Sojabohne |
| Gossypium hirsutum (Gossypium arboreum, Gossypium herbaceum, Gossypium vitifolium) | Baumwolle |
| Helianthus annuus | Sonnenblume |
| Helianthus tuberosus | Topinambur |
| Hevea brasiliensis | Parakautschukbaum |
| Hordeum vulgare | Gerste |
| Humulus lupulus | Hopfen |
| Ipomoea batatas | Süßkartoffeln |
| Juglans regia | Walnußbaum |
| Lactuca sativa | Kopfsalat |
| Lens culinaris | Linse |
| Linum usitatissimum | Faserlein |
| Lycopersicon lycopersicum | Tomate |
| Malus spp. | Apfel |
| Manihot esculenta | Maniok |
| Medicago sativa | Luzerne |
| Mentha piperita | Pfefferminze |
| Musa spp. | Obst- und Mehlbanane |
| Nicotiana tabacum (N. rustica) | Tabak |
| Olea europaea | Ölbaum |
| Oryza sativa | Reis |
| Panicum miliaceum | Rispenhirse |
| Phaseolus lunatus | Mondbohne |
| Phaseolus mungo | Erdbohne |
| Phaseolus vulgaris | Buschbohnen |
| Pennisetum glaucum | Perl- oder Rohrkolbenhirse |
| Petroselinum crispum spp. tuberosum | Wurzelpetersilie |
| Picea abies | Rotfichte |
| Abies alba | Weißtanne |
| Pinus spp. | Kiefer |
| Pisum sativum | Gartenerbse |
| Prunus avium | Süßkirsche |

| Botanischer Name | Deutscher Name |
|---|---|
| Prunus domestica | Pflaume |
| Prunus dulcis | Mandelbaum |
| Prunus persica | Pfirsich |
| Pyrus communis | Birne |
| Ribes sylvestre | Rote Johannisbeere |
| Ribes uva-crispa | Stachelbeere |
| Ricinus communis | Rizinus |
| Saccharum officinarum | Zuckerrohr |
| Secale cereale | Roggen |
| Sesamum indicum | Sesam |
| Solanum tuberosum | Kartoffel |
| Sorghum bicolor (s. vulgare) | Mohrenhirse |
| Sorghum dochna | Zuckerhirse |
| Spinacia oleracea | Spinat |
| Theobroma cacao | Kakaobaum |
| Trifolium pratense | Rotklee |
| Triticum aestivum | Weizen |
| Triticum durum | Hartweizen |
| Vaccinium corymbosum | Kulturheidelbeere |
| Vaccinium vitis-idaea | Preißelbeere |
| Vicia faba | Pferdebohnen |
| Vigna sinensis (V. unguiculata) | Kuhbohne |
| Vitis vinifera | Weinrebe |
| Zea mays | Mais |

Die erfindungsgemäßen 3-substituierten Pyridine können in diesen Anwendungsformen auch zusammen mit anderen Wirkstoffen vorliegen, z.B. mit Herbiziden, Insektiziden, Wachstumsregulatoren, Fungiziden oder auch mit Düngemitteln.

Beim Vermischen mit Fungiziden erhält man dabei in vielen Fällen eine Vergrößerung des fungiziden Wirkungsspektrums.

In der Anwendungsform als Herbizid kann durch Vermischen mit Herbiziden oder Wachstumsregulatoren in vielen Fällen das Wirkungsspektrum erweitert oder auch synergistische Effekte erzielt werden. Beispielsweise kommen als Mischungspartner Diazine, 4H-3,1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Cyclohexan-1,3-dionderivate, Chinolincarbonsäurederivate, Aryloxy-, Heteroaryloxyphenoxypropionsäuren sowie deren Salze, Ester und Amide und andere in Betracht.

Außerdem kann es von Nutzen sein, die 3-substituierten Pydridine I allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate zugesetzt werden.

Herstellungsbeispiele

Beispiel 1

1-(Pyrid-3-y])-2-(2-bromphenyl)-ethandiol

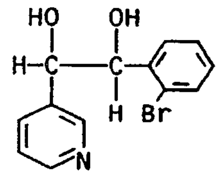

20 g (0,068 mol) 1-(Pyrid-3-yl)-2-(2-bromphenyl)-2-hydroxyethanon wurden in 300 ml Methanol bei 0 °C mit 3 g (0,079 mol) Natriumborhydrid reduziert. Man ließ einige Stunden bei Raumtemperatur (21 °C) nachrühren, stellte mit 4n Salzsäure auf pH 2 ein und gab 4 ml Ethylenglykol zu. Nach Einengen der Lösung wurde mit Essigester überschichtet und mit $NaHCO_3$-Lösung neutralisiert. Die Essigesterphase wurde zweimal mit Wasser gewaschen und über $Na_2SO_4$ getrocknet. Nach Abdestillieren des Essigesters fiel das Produkt als weißes Pulver an.
Schmelzpunkt: 115 °C.

Vorstufe 1.1
1-(Pyrid-3-yl )-2-(2-bromphenyl)-2-hydroxyethanon

146 g (0,8 mol) 2-Brombenzaldehyd, 85,6 g (0,8 mol) 3-Pyridylaldehyd, 20 g (0,08 mol) 3-Ethyl-5-(2-hydroxyethyl)-4-methylthiazoliumbromid und 40,4 g (0,4 mol) Triethylamin wurden in 500 ml Ethanol 16 Std. auf 75 °C erhitzt. Anschließend wurde das Ethanol abdestilliert und der Rückstand in Methylenchlorid aufgenommen. Nach zweifachem Waschen mit Wasser wurde zweimal mit 300 ml 4n Salzsäure extrahiert. Die saure wässrige Phase wurde anschließend mit 4n Natronlauge leicht alkalisch gestellt und mit Methylenchlorid dreifach extrahiert. Nach Abdestillieren des Lösungsmittels wurde das Rohprodukt mit Isopropanol gerührt, abgesaugt und getrocknet.
Schmelzpunkt: 105-108 °C.

Beispiel 2

3-(Pyrid-3-yl)-4-(2-chlorphenyl)-but-1-en-3,4-diol

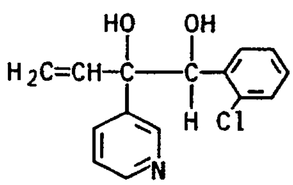

Eine Lösung von 13 g (0,0531 mol) 1-(Pyrid-3-yl)-2-(2-chlorphenyl)-2-hydroxyethanon in Tetrahydrofuran (THF) wurde bei Raumtemperatur zu einer frisch hergestellten Lösung von 0,18 mol Vinylmagnesiumbromid in 80 ml THF getropft. Nach drei Stunden Rühren bei Raumtemperatur wurde die Lösung durch Zugabe von Ammoniumchlorid-Lösung hydrolysiert. Die Wasserphase wurde zweimal mit Essigester extrahiert. Die gesammelten organischen Phasen wurden über $Na_2SO_4$ getrocknet, anschließend filtriert und eingeengt. Das Rohprodukt wurde mit Methyltert.-butylether gerührt, abgesaugt und getrocknet.
Schmelzpunkt: 110-112 °C.

Vorstufe 2.1
1-(Pyrid-3-yl)-2-(2-chlorphenyl)-2-hydroxyethanon

160 g (1,14 mol) 2-Chlorbenzaldehyd, 122 g (1,14 mol) 3-Pyridylaldehyd, 50 g (0,2 mol) 3-Ethyl-5-(2-hydroxyethyl)-4-methylthiazoliumbromid und 60,6 g (0,6 mol) Triethylamin wurden in einem Liter Ethanol 13 Std. auf 75 °C erhitzt. Anschließend wurde das Ethanol abdestilliert und der Rückstand in Methylenchlorid gelöst. Nach zweifachem Waschen mit Wasser wurde zweimal mit 300 ml 4n Salzsäure extrahiert. Die saure wässrige Phase wurde anschließend mit 4n Natronlauge leicht alkalisch gestellt und mit frischem Methylenchlorid dreifach extrahiert. Nach Abdestillieren des Lösungsmittels wurde das Rohprodukt mit Isopropanol gerührt, abgesaugt und getrocknet.

Schmelzpunkt: 92° C.

Beispiel 3

2-(Pyrid-3-yl)-4-methyl-butan-1,2-diol

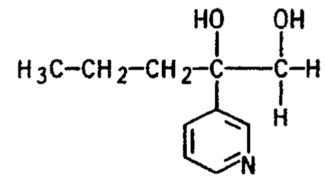

20 g (0,134 mol) 3-Pyridylisopropylketon und 30,2 g (0,268 mol) Kalium-tert.-butylat werden in 200 ml tert.-Butanol gelöst. Anschließend werden 37,4 g (0,208 mol) Trimethylsulfoniumjodid zugegeben und 1,5 Std. auf 65° C erwärmt. Nach Abtrennen der anorganischen Salze und Einengen der Reaktionslösung wird das Rohprodukt mit 75 ml halbkonz. $H_2SO_4$ versetzt und 8 Std. auf 60-70° C erwärmt. Danach wird mit 4n NaOH-Lösung auf pH 9 gestellt und mit Methylenchlorid extrahiert. Nach Trocknen und Einengen der organischen Phase wird das Produkt durch Chromatographie gereinigt.
$^1$H-NMR ($CDCl_3$/$TMS_{int}$): δ/ppm = 0.75(d,3H), 0.82(d,3H), 2.07(sept,1H),
3.4(s breit,1H), 3.85(d,1H), 4.08(d,1H),
7.1(m,1H), 7.8(m,1H), 8.35(m,1H), 8.52(d,1H)
IR: /cm$^{-1}$ = 3309, 3108, 2960, 1149, 1074, 1058, 908

Vorstufe 3.1
Pyrid-3-ylisopropylketon

133 g (0,70 mol) alpha-Morpholino-3-pyridylacetonitril, 25,7 g (0,07 mol) Tetrabutylammoniumiodid, 278 g (3,5 mol) 50 %ige NaOH, 250 ml (2,65 mol) Isopropylbromid und 300 ml Toluol wurden als Zweiphasensystem unter guter Durchmischung 4 Std. auf 50° C erwärmt. Die Aufarbeitung erfolgte durch Zugabe von Wasser, Phasentrennung und dreimaliges Waschen der organischen Phase mit Wasser. Nach Abdestillieren des Lösungsmittels wurde das Rohproduktöl bei 60° C in halbkonz. $H_2SO_4$ getropft. Nach 2 Std. wurde der Ansatz mit 50 %iger NaOH auf pH 9 gestellt und mit Toluol extrahiert. Die organische Phase wurde 3 mal mit Wasser gewaschen, die Waschlösung mit Toluol 4 mal extrahiert. Die gesammelten Toluolextrakte wurden über $Na_2SO_4$ getrocknet, abfiltriert und eingeengt. Das Produkt fiel als dunkel gefärbtes Öl an.
$^1$H-NMR ($CDCl_3$/$TMS_{int}$): δ/ppm = 1.25(d,6H), 3.55(sept,1H), 7.45(m,1H),
8.25(m,1H), 8.8(m,1H), 9.2(d,1H)

Beispiel 4

1-Acetoxy-1-(2,4-dichlorphenyl)-2-(pyrid-3-yl)-3-methyl-butan-2-ol

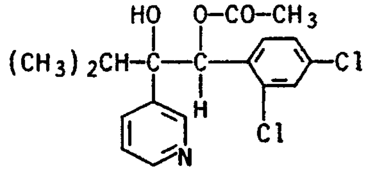

(Verbindung Nr. 1.01)

Zu einer Lösung aus 76 g (0,23 mol) 1-(2,4)-Dichlorphenyl-2-(3-pyridyl)-3-methyl-butan-1,2-diol, 197,7 ml (1,318 mol) Triethylamin und etwa 0,5 g (4 mmol) 4-N,N-Dimethylaminopyridin in 1 1 Methylenchlorid wurden unter Erwärmung auf 35° C 38,2 ml (0,4 mol) Acetanhydrid getropft. Nach Abkühlung auf Raumtemperatur wurde die Mischung noch 30 Minuten gerührt und dann in 500 ml einer gesättigten Natriumhydrogencarbonat-Lösung gegossen. Die organische Phase wurde abgetrennt und wie üblich auf

das Produkt hin aufgearbeitet. Das ölige Rohprodukt wurde durch Verrühren mit Essigester zur Kristallisation gebracht. Ausbeute: 56 %.

Beispiel 5

1-(2,4-Dichlorphenyl)-1-methylsulfonyloxy-2-(pyrid-3-yl)-octan-2-ol

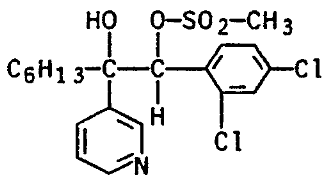

(Verbindung Nr. 2.04)

Zu einer Mischung aus 8,5 g (23 mmol) 1-(2,4-Dichlorphenyl)-2-(pyrid-3-yl)-octan-1,2-diol, 2,5 ml (32 mmol) Methansulfonylchlorid und 100 ml Methylenchlorid wurden bei etwa 20° C 23,6 ml (177 mmol) Triethylamin getropft. Anschließend wurde die Mischung mit 30 ml gesättigter Natriumhydrogencarbonat-Lösung und 30 ml Wasser gewaschen und wie üblich auf das Produkt hin aufgearbeitet. Die Reinigung des Rohproduktes erfolgte chromatographisch. Ausbeute 50,6 %.

Beispiel 6

1-(Pyrid-3-yl)-1-(4-fluorphenyl)-2-(2-chlorphenyl)-oxiran

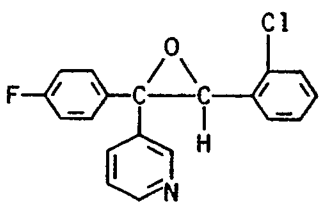

(Verbindung Nr. 3.16)

Zu einer Lösung aus 78,7 g (0,23 mol) 1-(Pyrid-3-yl)-1-(4-fluorphenyl)-2-(2-chlorphenyl)-ethandiol und 25,2 ml (0,32 mol) Methansulfonylchlorid in 1 1 Methylenchlorid wurden bei Rückflußtemperatur 236 ml (1,77 mol) Triethylamin getropft. Nach weiteren 4 Stunden Rühren bei Rückflußtemperatur wurde die Mischung mit 400 ml gesättigter Natriumhydrogencarbonat-Lösung und Wasser gewaschen und anschließend wie üblich auf das Produkt hin aufgearbeitet. Die Reinigung des öligen Rohproduktes erfolgte durch Flash-Chromatographie mit Cyclohexan/Essigester (8:3) als Laufmittel.
Ausbeute: 97 %; gelbes Öl.

Beispiel 7

2-Methoxy-2-methyl-4-(pyrid-3-yl)-4-isopropyl-5-(2,4-dichlorphenyl)-1,3-dioxolan

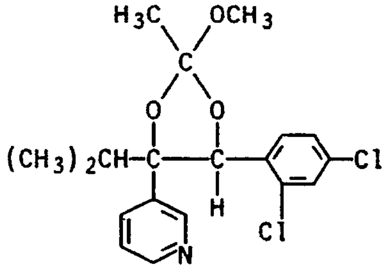

(Verbindung Nr. 4.01)

Eine Lösung aus 6,7 g (21 mmol) 1-(2,4-Dichlorphenyl)-2-(pyrid-3-yl)-3-methyl-butan-1,2-diol und etwa 0,3 g (1,7 mmol) p-Toluolsulfonsäure in 50 ml Orthoessigsäuremethylester wurde 1 Stunde bei 60 °C gerührt. Nachdem man überschüssigen Orthoessigsäuremethylester und das gebildete Methanol langsam im Vakuum entfernt hatte, wurde der Rückstand in 50 ml Methylenchlorid gelöst. Die organische Phase wurde mit gesättigter Natriumhydrogencarbonat-Lösung gewaschen und wie üblich auf das Produkt hin aufgearbeitet. Die Reinigung des Rohproduktes erfolgte durch Flash-Chromatographie mit Essigester/Cyclohexan (3:7) als Laufmittel. Ausbeute: 49 %.

Beispiel 8

2-Phenyl-4-(pyrid-3-yl)-5-(2-bromphenyl)-1,3-dioxolan

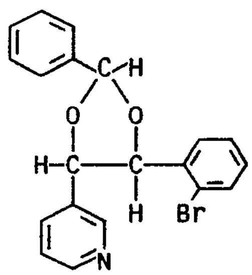

(Verbindung Nr. 5.69)

37 g (0,1258 mol) 1-(Pyrid-3-yl)-2-(2-bromphenyl)-ethandiol und 38,5 g (0,2516 mol) Benzaldehyddimethylacetal wurden in 440 ml Chlorbenzol/Toluol (10:1) zusammen mit 1 g p-Toluolsulfonsäure zum Rückfluß erhitzt. Das entstehende Methanol wurde als Azeotrop mit Toluol abdestilliert. Nach beendeter Reaktion wurde das Chlorbenzol unter vermindertem Druck abdestilliert und das Produkt durch Chromatographie an Kieselgel gereinigt.

Beispiel 9

2-tert.-Butyl-4-(pyrid-3-yl)-5-(2-bromphenyl)-1,3-dioxolan

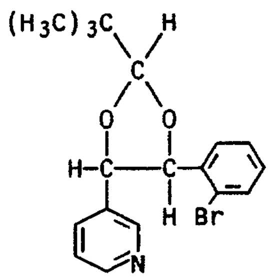

(Verbindung Nr. 5.70)

8 g (0,027 mol) 1-(Pyrid-3-yl)-2-(2-bromphenyl)-ethandiol und 4,5 g (0,0525 mol) Pivaldehyd wurden in 100 ml Methylenchlorid gelöst. Dazu tropfte man bei Raumtemperatur 4,35 ml (0,0525 mol) Bortrifluoridetherat und rührte über Nacht nach. Anschließend wurde auf Eiswasser gegeben und mit $NaHCO_3$-Lösung neutral gestellt. Die organische Phase wurde mit Wasser gewaschen, über $Na_2SO_4$ getrocknet, abfiltriert und eingeengt. Das Rohprodukt wurde durch Chromatographie gereinigt.

$^1$H-NMR ($CDCl_3$/$TMS_{int}$): δ/ppm = 1.2(s,9H), 4.85(s,1H), 5.5(d,1H), 5.7(d,1H), 6.9-7.5(m,6H), 8.25(m,1H), 8.5(dm1H)

Beispiel 10

2-Phenyl-4-(pyrid-3-yl)-4-ethenyl-5-(2-chlorphenyl)-1,3-dioxolan

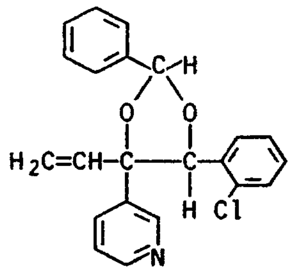

(Verbindung Nr. 5.71)

3 g (0,018 mol) 3-(Pyrid-3-yl)-4-(2-chlorphenyl)-but-1-en-3,4-diol wurden in 50 ml Benzaldehyddimethylacetal gelöst. Anschließend wurde 1 ml konz. $H_2SO_4$ zugegeben und auf 80-90 °C erhitzt. Das überschüssige Benzaldehyddimethylacetal wurde zusammen mit dem entstehenden Methanol im Vakuum abdestilliert. Das Rohprodukt wurde in Methylenchlorid gelöst und mit $NaHCO_3$-Lsg. neutralisiert. Nach Abtrennen des Lösungsmittels wurde das Produkt durch Chromatographie gereinigt.

Die physikalischen Daten der Endprodukte I sind den folgenden Tabellen 1 bis 5 zu entnehmen, in denen noch weitere Verbindungen I aufgeführt sind, welche auf die gleichen Weisen hergestellt wurden oder herstellbar sind.

Tabelle 1

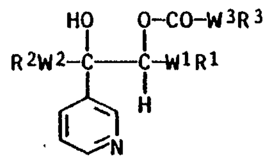

I (X= OH; Y= –O–CO–$W^3R^3$)

| Nr. | $W^1R^1$ | $W^2R^2$ | $W^3R^3$ | Physik. Daten Fp. [°C]; $^1H$-NMR*) [ppm] |
|---|---|---|---|---|
| 1.01 | 2,4-Dichlorphenyl | iso-Propyl | Methyl | 162-167 |
| 1.02 | 2,4-Dichlorphenyl | Methyl | Methyl | |
| 1.03 | 2,4-Dichlorphenyl | iso-Propyl | Ethyl | 110-112 |
| 1.04 | 2,4-Dichlorphenyl | iso-Propyl | Phenyl | 215-219 |
| 1.05 | 2,4-Dichlorphenyl | iso-Propyl | n-Propyl | 120-123 |
| 1.06 | 2,4-Dichlorphenyl | iso-Propyl | 4-Fluorbenzyl | |
| 1.07 | 2,4-Dichlorphenyl | 4-Fluorphenyl | Methyl | |
| 1.08 | 2,4-Dichlorphenyl | 4-Fluorbenzyl | t-Butyl | |
| 1.09 | 2,4-Dichlorphenyl | iso-Propyl | Wasserstoff | |
| 1.10 | 2,4-Dichlorphenyl | iso-Propyl | iso-Propyl | 140-143 |
| 1.11 | 2,4-Dichlorphenyl | Vinyl | Methyl | |
| 1.12 | 2,4-Dichlorphenyl | Allyl | Methyl | |
| 1.13 | 2-Methoxyphenyl | Phenyl | Methylthiomethyl | |
| 1.14 | 3-Chlorthien-3-yl | Butyl | Methyl | |
| 1.15 | 3-Isopropylisoxazol-5-yl | 2,4-Dichlorphenyl | 4-Chlorphenyl | |

*) in $CDCl_3$, TMS als interner Standard

Tabelle 2

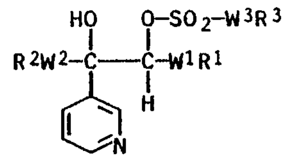

I (X= OH; Y= $-O-SO_2-W^3R^3$)

| Nr. | $W^1R^1$ | $W^2R^2$ | $W^3R^3$ | Physik. Daten Fp. [°C]; $^1$H-NMR*) [ppm] |
|---|---|---|---|---|
| 2.01 | 2,4-Dichlorphenyl | iso-Propyl | Methyl | 138-140 |
| 2.02 | 2,4-Dichlorphenyl | Methyl | Methyl | 155-160 |
| 2.03 | 2,4-Dichlorphenyl | n-Butyl | Methyl | Öl, NMR: 0.87(t, 3H), 2.72(s, 3H), 6.1(s, 1H) |
| 2.04 | 2,4-Dichlorphenyl | n-Hexyl | Methyl | 60-67 |
| 2.05 | 2,4-Dichlorphenyl | iso-Propyl | Phenyl | |
| 2.06 | 2,4-Dichlorphenyl | iso-Propyl | 4-Methylphenyl | |
| 2.07 | 2,4-Dichlorphenyl | 4-Fluorphenyl | Methyl | |
| 2.08 | 2,4-Dichlorphenyl | 4-Fluorbenzyl | Phenyl | |
| 2.09 | 2,4-Dichlorphenyl | iso-Propyl | 4-Chlorphenyl | |
| 2.10 | 2,4-Dichlorphenyl | iso-Propyl | n-Butyl | |
| 2.11 | 2,4-Dichlorphenyl | Vinyl | Methyl | |
| 2.12 | 2,4-Dichlorphenyl | Allyl | Methyl | |
| 2.13 | 2-Methoxyphenyl | Phenyl | 4-Methylphenyl | |

*) in $CDCl_3$, TMS als interner Standard

Tabelle 2 (Fortsetzung)

| Nr. | $W^1R^1$ | $W^2R^2$ | $W^3R^3$ | Physik. Daten Fp. [°C]; $^1$H-NMR*) [ppm] |
|---|---|---|---|---|
| 2.14 | 3-Chlorthien-3-yl | Butyl | Methyl | |
| 2.15 | 3-Isopropylisoxazol-5-yl | 2,4-Dichlorphenyl | 4-Chlorphenyl | |
| 2.16 | 2,4-Dichlorphenyl | 4-Chlorphenethyl | Methyl | 113-115 |
| 2.17 | 2,4-Dichlorphenyl | 4-Fluorphenethyl | Methyl | 67-70 |

*) in $CDCl_3$, TMS als interner Standard

Tabelle 3

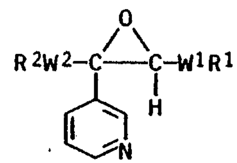

I (X+Y= Sauerstoff)

| Nr. | $W^1R^1$ | $W^2R^2$ | Physik. Daten Fp. [°C]; $^1$H-NMR*) [ppm] |
|---|---|---|---|
| 3.01 | 2,4-Dichlorphenyl | iso-Propyl | Öl,NMR:0.74(d,3H), 1.42(m,1H),4.13(s,1H) |
| 3.02 | 2,4-Dichlorphenyl | Methyl | 92-103 |
| 3.03 | 2,4-Dichlorphenyl | n-Butyl | Öl,NMR;0,75(t,3H),4.0(2,1H) |
| 3.04 | 2,4-Dichlorphenyl | n-Hexyl | Öl,NMR:0.8(t,3H),4.0(s,1H) |
| 3.05 | 2,4-Dichlorphenyl | Benzyl | 140 |
| 3.06 | 2-Bromphenyl | Benzyl | 105-110 |
| 3.07 | 2-Bromphenyl | 2-Chlorbenzyl | 105- 107 |
| 3.08 | 2-Bromphenyl | 4-Chlorbenzyl | 77 |
| 3.09 | 2,4-Dichlorphenyl | 4-Chlorbenzyl | 113 |
| 3.10 | 2,4-Dichlorphenyl | 2-Chlorbenzyl | 110- 112 |
| 3.11 | 2,4-Dichlorphenyl | 2,4-Dichlorbenzyl | 126- 128 |
| 3.12 | 2,4-Dichlorphenyl | 4-Fluorbenzyl | 122- 124 |
| 3.13 | 2,4-Dichlorphenyl | 4-Fluorphenyl | 94-98 |
| 3.14 | 2,4-Dichlorphenyl | 4-Chlorphenethyl | Öl,NMR:1.6(m,1H),2.18(m,1H), 2.35(m,2H),4.02(s,1H) |

*) in $CDCl_3$, TMS als interner Standard

Tabelle 3 (Fortsetzung)

| Nr. | $W^1R^1$ | $W^2R^2$ | Physik. Daten Fp. [°C]; $^1H$-NMR*) [ppm] |
|---|---|---|---|
| 3.15 | 3-Isopropylisoxazol-5-yl | 2,4-Dichlorphenyl | |
| 3.16 | 2-Chlorphenyl | 4-Fluorphenyl | Öl, NMR: 4.65(s, 1H), 6.9(t, 2H), 7.0-7.38(m, 8H), 7.72-7.82(m, 1H), 8.55-8.63(m, 1H), 8.78(d, 1H) |
| 3.17 | 2,4-Dichlorphenyl | Phenyl | |
| 3.18 | 2,4-Dichlorphenyl | 4-Chlorphenyl | 110-115 |
| 3.19 | 2,4-Dichlorphenyl | 4-Fluorphenethyl | Öl, NMR: 2.35(m, 2H), 4.03(s, 1H) |
| 3.20 | 2,4-Dichlorphenyl | Vinyl | Öl, NMR: 4.02(s, 1H), 5.1-33(m, 2H), 5.55-72(m, 1H) |
| 3.21 | 2,4-Dichlorphenyl | Allyl | |
| 3.22 | 2-Chlorthien-3-yl | iso-Propyl | |
| 3.23 | 2-Methoxyphenyl | iso-Propyl | |

*) in $CDCl_3$, TMS als interner Standard

Tabelle 4

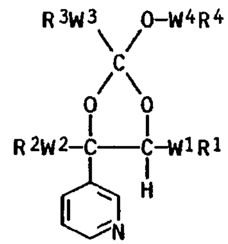

I 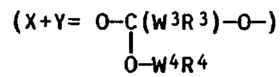

| Nr. | $W^1R^1$ | $W^2R^2$ | $W^3R^3$ | $W^4R^4$ | Physik. Daten<br>Fp. [°C]; $^1H$-NMR*) [ppm] |
|---|---|---|---|---|---|
| 4.01 | 2,4-Dichlorphenyl | iso-Propyl | Methyl | Methyl | Öl,NMR:0.75(d,3H),1.4(d,3H),2,55(m,1H),<br>3.47(s,3H),6.0(s,1H),6.45-8.4(m,7H),<br>1.92(s,3H),3.47(s,3H),6.0(s,1H) |
| 4.02 | 2,4-Dichlorphenyl | Methyl | Methyl | Methyl | Öl,NMR:1.9(s,3H),2.08(s,3H),<br>3.46(s,3H),5.83(s,1H) |
| 4.03 | 2,4-Dichlorphenyl | n-Butyl | Methyl | Methyl | Öl,NMR:1.9(s,3H),3.46(s,3H)<br>5.83(s,3H) |
| 4.04 | 2,4-Dichlorphenyl | n-Hexyl | Methyl | Methyl | Öl,NMR:1.9(s,3H),3.48(s,3H)<br>5.93(s,1H) |
| 4.05 | 2,4-Dichlorphenyl | iso-Propyl | Phenyl | Ethyl | |
| 4.06 | 2,4-Dichlorphenyl | iso-Propyl | 4-Methylphenyl | Methyl | |
| 4.07 | 2,4-Dichlorphenyl | 4-Fluorphenyl | Methyl | Methyl | Öl,NMR:1.93(s,3H),3.2(s,3H),<br>6.54(s,1H) |
| 4.08 | 2,4-Dichlorphenyl | 4-Fluorphenyl | Methyl | Methyl | Öl,NMR:1.92(s,3H),3.52(s,3H),<br>5.95(s,3H) |

*) in $CDCl_3$, TMS als interner Standard

Tabelle 4 (Fortsetzung)

| Nr. | $W^1R^1$ | $W^2R^2$ | $W^3R^3$ | $W^4R^4$ | Physik. Daten Fp. [°C]; $^1$H-NMR*) [ppm] |
|---|---|---|---|---|---|
| 4.09 | 2,4-Dichlorphenyl | iso-Propyl | 4-Chlorphenyl | Methyl | |
| 4.10 | 2,4-Dichlorphenyl | iso-Propyl | n-Butyl | Methyl | |
| 4.11 | 2,4-Dichlorphenyl | Vinyl | Methyl | Methyl | 105-108 |
| 4.12 | 2,4-Dichlorphenyl | Allyl | Methyl | Methyl | |
| 4.13 | 2-Methoxyphenyl | Phenyl | 4-Methylphenyl | Methyl | |
| 4.14 | 2-Chlorthien-3-yl | iso-Propyl | Methyl | Methyl | |
| 4.15 | 3-Isopropyl-isoxazol-5-yl | 2,4-Dichlorphenyl | 4-Chlorphenyl | Methyl | |
| 4.16 | 2,4-Dichlorphenyl | Benzyl | Methyl | Methyl | 155 |
| 4.17 | 2-Bromphenyl | Benzyl | Methyl | Methyl | 130-135 |
| 4.18 | 2-Chlorphenyl | 4-Fluorphenyl | Methyl | Methyl | Öl,NMR:1.93(s,3H),3.2(s,3H), 6.6(s,1H) |
| 4.19 | 2-Methoxyphenyl | 4-Fluorphenyl | Methyl | Methyl | 117-124 |
| 4.20 | 2-Fluorphenyl | 4-Fluorphenyl | Methyl | Methyl | Öl,NMR:1.95(s,3H),3.17(s,3H), 6.52(s,1H) |
| 4.21 | 2-Methoxyphenyl | 4-Fluorbenzyl | Methyl | Methyl | Öl,NMR:1.92(s,3H),3.48(s,3H), 5.95(s,1H) |
| 4.22 | 2-Chlorphenyl | 4-Fluorbenzyl | Methyl | Methyl | Öl,NMR:1.92(s,3H),3.53(s,3H), 6.05(s,1H) |
| 4.23 | 2-Fluorphenyl | 4-Fluorbenzyl | Methyl | Methyl | Öl,NMR:1.92(s,3H),3.48(s,3H), 5.88(s,1H) |
| 4.24 | 2,4-Dichlorphenyl | 2,4-Dichlorbenzyl | Wasserstoff | Methyl | 119-123 |

*) in $CDCl_3$, TMS als interner Standard

Tabelle 4 (Fortsetzung)

| Nr. | $W^1R^1$ | $W^2R^2$ | $W^3R^3$ | $W^4R^4$ | Physik. Daten Fp. [°C]; $^1H$-NMR*) [ppm] |
|---|---|---|---|---|---|
| 4.25 | 2,4-Dichlorphenyl | Phenyl | Methyl | Methyl | Öl,NMR:1.93(s,3H),3.18(s,3H), 6.6(s,1H) |
| 4.26 | 2,4-Dichlorphenyl | 4-Chlorphenyl | Methyl | Methyl | Öl,NMR:1.93(s,3H),3.2(s,3H), 6.53(s,1H) |
| 4.27 | 2,4-Dichlorphenyl | 4-Fluorphen-ethyl | Methyl | Methyl | Öl,NMR:1.96(s,3H),3.5(s,3H), 5.88(s,1H) |
| 4.28 | 2,4-Dichlorphenyl | 4-Chlorbenzyl | Methyl | Methyl | 123-134 |
| 4.29 | 2,4-Dichlorphenyl | 2-Chlorbenzyl | Methyl | Methyl | Öl,NMR:1.96(s,3H),3.48(s,3H), 6.0(s,1H) |
| 4.30 | 2,4-Dichlorphenyl | 4-Chlorphen-ethyl | Methyl | Methyl | Öl,NMR:1.93(s,3H),3.47(s,3H), 5.88(s,1H) |

*) in $CDCl_3$, TMS als interner Standard

Tabelle 5

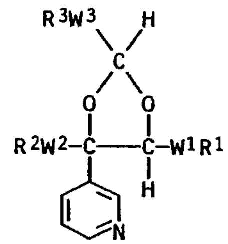

I (X+Y = $-O-\underset{H}{C}(W^3R^3)-O-$)

| Nr. | $W^1R^1$ | $W^2R^2$ | $W^3R^3$ | Phys. Daten (Fp. [°C]; IR [$cm^{-1}$]; $^1H$-NMR*) [ppm]) |
|---|---|---|---|---|
| 5.01 | 4-Fluorphenyl | Wasserstoff | Phenyl | Öl,NMR:6.2(s,1H),5.58(d,1H)5.52(d,1H) |
| 5.02 | 2-Chlorphenyl | Wasserstoff | Phenyl | Öl,NMR:6.2(s,1H),5.9(d,1H),5.72(d,1H) |
| 5.03 | 2,4-Dichlorphenyl | Wasserstoff | Phenyl | 93-95 |
| 5.04 | 2,4-Dichlorphenyl | Wasserstoff | 4-Methylphenyl | Öl,NMR:6.18(s,1H),5.82(d,1H),5.7(d,1H) |
| 5.05 | 2,4-Dichlorphenyl | Wasserstoff | 4-Fluorphenyl | 123-125 |
| 5.06 | 2-Chlorphenyl | Wasserstoff | 4-Methylphenyl | Öl,NMR:6.18(s,1H),5.85(d,1H),5.7(d,1H) |
| 5.07 | 2-Chlorphenyl | Wasserstoff | 4-Fluorphenyl | Öl,NMR:6.2(s,1H),5.87(d,1H),5.75(d,1H) |
| 5.08 | 2,4-Dichlorphenyl | Wasserstoff | t-Butyl | Öl,NMR:5.6(d,1H),5.4(d,1H),4.8(s,1H) 1.2(s.9H) |
| 5.09 | 2,4-Dichlorphenyl | Wasserstoff | 4-Fluorbenzyl | Öl,NMR:5.6(d,1H),5.45(d,1H),5.4(t,1H) 3.3(d,1H) |
| 5.10 | 2-Methoxyphenyl | Wasserstoff | Phenyl | 110-114 |
| 5.11 | 2-Fluorphenyl | Wasserstoff | Phenyl | 72-75 |
| 5.12 | 2-Methylphenyl | Wasserstoff | Phenyl | 72-75 |

*) in $CDCl_3$; TMS als interner Standard

| Nr. | $W^1R^1$ | $W^2R^2$ | $W^3R^3$ | Phys. Daten (Fp. [°C]; IR [$cm^{-1}$]; $^1H$-NMR*) [ppm]) |
|---|---|---|---|---|
| 5.13 | 2-Bromphenyl | Wasserstoff | 4-Fluorphenyl | 90-93 |
| 5.14 | 2-Bromphenyl | Wasserstoff | 4-Methylphenyl | öl, NMR:6.18(s,1H),5.72(d,1H),5.85(d,1H) |
| 5.15 | 2-Chlorphenyl | Wasserstoff | 4-t-Butylphenyl | 125-130 |
| 5.16 | 2-Bromphenyl | Wasserstoff | 4-t-Butylphenyl | 145-150 |
| 5.17 | 2-Chlorphenyl | Wasserstoff | t-Butyl | öl, IR:1482,1435,1100,1054,1040,990,771, 760,717 |
| 5.18 | 2,4-Dichlorphenyl | Phenyl | Phenyl | 160 |
| 5.19 | 2,4-Dichlorphenyl | 4-Fluorphenyl | Phenyl | 180-183 |
| 5.20 | 2-Bromphenyl | Benzyl | Phenyl | 132-135 |
| 5.21 | 2-Bromphenyl | 2-Chlorphenyl | Phenyl | 127-130 |
| 5.22 | 2-Bromphenyl | 4-Chlorphenyl | Phenyl | öl, IR:1490,1417,1090,1062.1026,1017,747, 737,697 |
| 5.23 | 2,4-Dichlorphenyl | 4-Fluorbenzyl | Phenyl | 155-157 |
| 5.24 | 2,4-Dichlorphenyl | 2,4-Dichlor-benzyl | Phenyl | öl, IR:1474,1103,1091,1042,1028,991,757, 712 |
| 5.25 | 2,4-Dichlorphenyl | Ethenyl | Phenyl | öl, IR:1474,1091,1069,1053,1027,755,711, 698 |
| 5.26 | 2-Bromphenyl | Ethenyl | Phenyl | öl, NMR:5.9(s,1H),6.25(s,1H) |
| 5.27 | 2,4-Dichlorphenyl | Propen-3-yl | Phenyl | öl, IR:1477,1418,1091,1069,1039,1028,987, 761,714 |
| 5.28 | 2,4-Dichlorphenyl | iso-Propyl | Phenyl | |
| 5.29 | 2,4-Dichlorphenyl | Cyclopropyl | Phenyl | |
| 5.30 | 2-Bromphenyl | Cyclohexyl | Phenyl | |
| 5.31 | 2-Bromphenyl | 3-Pyridyl | Phenyl | |
| 5.32 | 2-Cyclophenyl | 3-Pyridyl | Phenyl | |
| 5.33 | 2-Chlorphenyl | iso-Propyl | Phenyl | |

*) in $CDCl_3$; TMS als interner Standard

| Nr. | $W^1R^1$ | $W^2R^2$ | $W^3R^3$ | Phys. Daten (Fp. [°C]; IR [$cm^{-1}$]; $^1H$-NMR*) [ppm]) |
|---|---|---|---|---|
| 5.34 | 2-Chlorthienyl | Wasserstoff | Phenyl | |
| 5.35 | 3-Bromthien-2-yl | Wasserstoff | Phenyl | |
| 5.36 | 3-Isopropyl-oxazol-5-yl | Wasserstoff | Phenyl | |
| 5.37 | 3-Phenyliso-xazol-5-yl | Wasserstoff | Phenyl | |
| 5.38 | 2-Brom-4-fluor-phenyl | Wasserstoff | Phenyl | |
| 5.39 | 2-Chlor-4-fluor-phenyl | Wasserstoff | Phenyl | |
| 5.40 | Wasserstoff | iso-Propyl | Phenyl | |
| 5.41 | Wasserstoff | iso-Propyl | 4-Fluorphenyl | |
| 5.42 | Wasserstoff | iso-Propyl | 4-Methylphenyl | |
| 5.43 | Wasserstoff | iso-Propyl | 4-Chlorphenyl | |
| 5.44 | Wasserstoff | iso-Propyl | 4-Methoxyphenyl | |
| 5.45 | 4-Cyanophenyl | Wasserstoff | Phenyl | |
| 5.46 | 4-Methoximino | Wasserstoff | Phenyl | |
| 5.47 | 2-Bromphenyl | Wasserstoff | Methyl | |
| 5.48 | 2-Bromphenyl | Wasserstoff | Chlormethyl | |
| 5.49 | 2-Bromphenyl | Wasserstoff | Methylthiomethyl | |
| 5.50 | 2-Bromphenyl | Wasserstoff | Methoxymethyl | |
| 5.51 | 2-Bromphenyl | Wasserstoff | Trichlormethyl | |
| 5.52 | iso-Propyl | Wasserstoff | Phenyl | |
| 5.53 | Methyl | Wasserstoff | Phenyl | |
| 5.54 | Cyclohexyl | Wasserstoff | Phenyl | |
| 5.55 | Pentyl | 4-Fluorbenzyl | Phenyl | |

*) in $CDCl_3$; TMS als interner Standard

| Nr. | $W^1R^1$ | $W^2R^2$ | $W^3R^3$ | Phys. Daten (Fp. [°C]; IR [$cm^{-1}$]; $^1$H-NMR*) [ppm]) |
|---|---|---|---|---|
| 5.56 | iso-Propyl | 4-Fluorbenzyl | Phenyl | |
| 5.57 | iso-Propyl | 4-Fluorbenzyl | Phenyl | |
| 5.58 | iso-Propyl | Wasserstoff | 4-Fluorphenyl | |
| 5.59 | t-Butyl | Wasserstoff | Phenyl | |
| 5.60 | t-Butyl | Wasserstoff | 4-Methoximinophenyl | |
| 5.61 | 2-Chlorphenyl | Propinyl | Phenyl | |
| 5.62 | 2-Chlorphenyl | 2-Pyridyl | Phenyl | |
| 5.63 | 2-Chlorphenyl | Wasserstoff | 4-Methylcyclohexyl | |
| 5.64 | 2-Trifluormethyl | Wasserstoff | Phenyl | |
| 5.65 | 2-Bromphenyl | Wasserstoff | 1-Naphthyl | |
| 5.66 | 2-Chlorphenyl | 2-(4-Chlor-phenyl)eth-1-yl | Phenyl | |
| 5.67 | 2-Chlorphenyl | 1-(4-Fluor-phenyl)eth-1-yl | Phenyl | |
| 5.68 | 2-Bromphenyl | Wasserstoff | 2-Nitrophenyl | |
| 5.69 | 2-Bromphenyl | Wasserstoff | Phenyl | NMR: 5.75 (d,1H), 5.9 (d,1H), 6.2 (s,1H), 6.9-8.45 (m,1H) |
| 5.70 | 2-Bromphenyl | Wasserstoff | t-Butyl | 100-103 |
| 5.71 | 2-Chlorphenyl | Ethenyl | Phenyl | NMR: 5.55 (d,1H), 5.75 (d,1H), 5.9 (s,1H), 6.2 (s,1H), 6.65 (dd,1H), 6.9-8.5 (m,13H) |

*) in $CDCl_3$; TMS als interner Standard

Die N-Oxide der 3-substituierten Pyridine I lassen sich beispielsweise dadurch herstellen, daß man die Verbindungen I mit einer Persäure wie meta-Chlorperbenzoesäure oder Peressigsäure behandelt und anschließend mit wäßriger $NaHCO_3$-Lösung die entstandene Carbonsäure abtrennt.

Beispiel 11

2-Phenyl-4-(pyrid-3-yl-N-Oxid)-5-(2-methoxyphenyl)-1,3-dioxolan

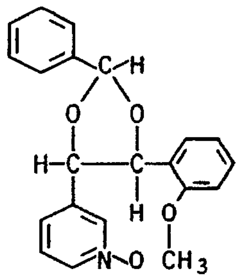

(Verbindung Nr. 5.72)

In 150 ml $CH_2Cl_2$ wurden 4,7 g (0,022 mol) 80 %ige meta-Chlorperbenzoesäure vorgelegt. Dazu tropfte man eine Lösung von 6,7 g (0,019 mol) 2-Phenyl-4-(3-pyridyl)5-(2-methoxyphenyl)-1,3-dioxolan in 50 ml $CH_2Cl_2$ und ließ ca. 8 Stunden bei Raumtemperatur nachrühren. Die ausgefallene meta-Chlorbenzoesäure wurde abfiltriert, die organische Phase je zweimal mit $NaHCO_3$-Lösung und $NaHSO_3$-Lösung gewaschen und anschließend über $Na_2SO_4$ getrocknet. Nach Einengen des Lösungsmittels verblieb ein braunes Öl.

$^1$H-NMR ($CDCl_3$/$TMS_{int}$): $\delta$/ppm = 3.8 (s, 3H), 5.5 (d,1H), 5.75 (d,1H), 6.15 (s,1H), 6.6-8.1 (m,13H)

Anwendungsbeispiele (fungizide Wirksamkeit)

Als Vergleichssubstanzen dienten

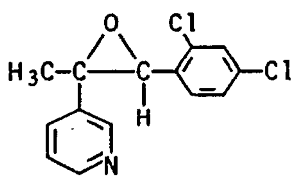

A,

bekannt aus der EP-A 074 018 (Beispiel 9)
und

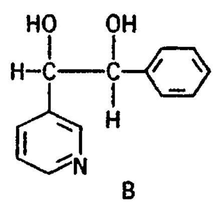

B

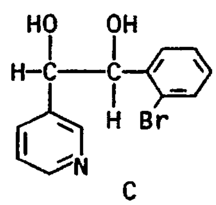

C

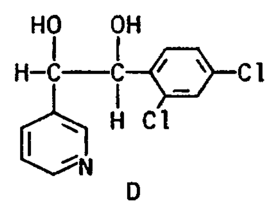

D

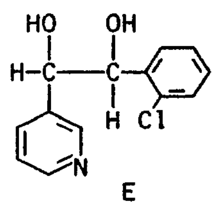

E

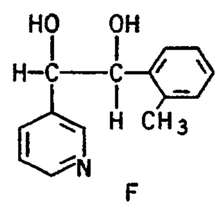

F

bekannt aus Tetrahedron 24, 1959 (1968).

Beispiel 12

Wirksamkeit gegen Botrytis cinerea

Paprikasämlinge der Sorte "Neusiedler Ideal Elite" wurden, nachdem sich 4 bis 5 Blätter gut entwickelt hatten, mit wäßrigen Suspensionen, die 80 % Wirkstoff und 20 % Emulgiermittel in der Trockensubstanz enthielten, tropfnaß gespritzt. Nach dem Antrocknen des Spritzbelages wurden die Pflanzen mit einer Konidienaufschwemmung des Pilzes Botrytis cinerea besprüht und bei 22 bis 24° C in eine Kammer mit 90 bis 95 % relativer Luftfeuchtigkeit gestellt. Nach 5 Tagen hatte sich die Krankheit auf unbehandelten Kontrollpflanzen so stark entwickelt, daß die entstandenen Blattnekrosen den überwiegenden Teil der Blätter bedeckten.

Das Ergebnis aus zwei unabhängigen Versuchen zeigt, daß die Wirkstoffe 1.01, 2.03, 2.04, 3.06, 4.01 und 4.11 sowie 5.6, 5.7, 5.14 und 5.69 bei der Anwendung als 0,05 %ige (Gew.-%) Spritzbrühe eine gute fungizide Wirkung gegen Botrytis cinerea aufweisen (83 % und 80 %), während die bekannten Vergleichsverbindungen A, C und E keine fungizide Wirkung (0 %) aufweisen.

Beispiel 13

Wirksamkeit gegen Weizenbraunrost

Blätter von in Töpfen gewachsenen Weizensämlingen der Sorte "Kanzler" wurden mit Sporen des Braunrostes (Puccinia recondita) bestäubt. Danach wurden die Töpfe für 24 Stunden bei 20 bis 22° C in eine Kammer mit 90 bis 95 % relativer Luftfeuchtigkeit gestellt. Während dieser Zeit keimten die Sporen aus und die Keimschläuche drangen in das Blattgewebe ein. Die infizierten Pflanzen wurden mit 0,025 %iger wäßriger Spritzbrühe, die 80 % Wirkstoff und 20 % Emulgiermittel in der Trockensubstanz enthielten, tropfnaß gespritzt und nach dem Antrocknen des Spritzbelages im Gewächshaus bei einer Temperatur zwischen 20 und 22° C und 65 bis 70 % relativer Luftfeuchtigkeit aufgestellt. Nach 8 Tagen beurteilte man das Ausmaß der Rostpilzentwicklung auf den Blättern.

Das Ergebnis zeigt, daß die Wirkstoffe 1.01, 4.01, 4.02, 4.03, 4.04, 4.07, 4.08, 4.11, 4.20, 4.22, 4.25, 4.26 und 4.27 bei der Anwendung als 0,025 %ige (Gew.-%) Spritzbrühe eine sehr gute fungizide Wirkung gegen den Braunrost aufweisen (98 %), während die bekannte Vergleichsverbindung A keine fungizide Wirkung hat.

Beispiel 14

Wirksamkeit gegen Gurkenmehltau (Dauer)

Junge Gurkenkeimlinge der Sorte "Chinesische Schlange" wurden im Zweiblattstadium mit einer Sporensuspension des Gurkenmehltaus (Erysiphe cichoracearum und Sphaerotheca fuliginea) besprüht. Nach etwa 20 Stunden wurden die Versuchspflanzen mit wäßriger Spritzbrühe, die 80 % Wirkstoff und 20 % Emulgiermittel in der Trockensubstanz enthielt, bis zur Tropfnäße besprüht. Nach dem Antrocknen des Spritzbelages stellte man die Pflanzen im Gewächshaus bei Temperaturen zwischen 20 und 22° C und 70 bis 80 % relativer Luftfeuchtigkeit auf. Das Ausmaß der Pilzentwicklung wurde nach 21 Tagen ermittelt.

Das Ergebnis aus zwei unabhängigen Versuchen zeigt, daß die Wirkstoffe 1.01, 1.03, 2.03, 3.13, 3.18, 4.01, 4.02, 4.03, 4.04, 4.07, 4.08, 4.11, 4,18, 4.25, 4.27 und 4.30 sowie 5.6, 5.7, 5.9, 5.13, 5.14, 5.69 und 5.70 bei der Anwendung als 0,025 %ige Spritzbrühe eine bessere fungizide Wirkung aufweisen (97 % und 90 %) als die bekannten Vergleichswirkstoffe A (50 % ) sowie C, D und E (10 %).

Beispiel 15

Wirksamkeit gegen Pyrenophora teres

Gerstenkeimlinge der Sorte "Igri" wurden im Zweiblattstadium mit wäßrigen Suspensionen, die 80 % Wirkstoff (Gew.-%) und 20 % Emulgator in der Trockensubstanz enthielten, tropfnaß gespritzt. Nach 24 Stunden werden die Pflanzen mit einer Sporensuspension des Pilzes Pyrenophora teres inokuliert und für 48 Stunden in eine Klimakammer mit hoher Luftfeuchtigkeit bei 18° C gestellt. Anschließend werden die Pflanzen im Gewächshaus bei 20 bis 22° c und 70 % relativer Luftfeuchtigkeit für weitere 5 Tage kultiviert. Dann wird das Ausmaß des Blattbefalls ermittelt.

Das Ergebnis zeigt, daß die Wirkstoffe 5.2, 5.3, 5.6, 5.7, 5.12, 5.13, 5.14 und 5.69 bei der Anwendung als 0,05 %ige Spritzbrühe eine bessere fungizide Wirkung zeigen (90 %) als die bekannten Vergleichswirkstoffe C, D, E und F (10 %).

Anwenidungsbeispiele (herbizide Wirksamkeit)

Die herbizide Wirkung der 3-substituierten Pyridine der Formel I ließ sich durch Gewächshausversuche zeigen:

Als Kulturgefäße dienten Plastikblumentöpfe mit lehmigem Sand mit etwa 3,0 % Humus als Substrat. Die Samen der Testpflanzen wurden nach Arten getrennt eingesät.

Bei Vorauflaufbehandlung wurden die in Wasser suspendierten oder emulgierten Wirkstoffe direkt nach Einsaat mittels fein verteilender Düsen aufgebracht. Die Gefäße wurden leicht beregnet, um Keimung und Wachstum zu fördern und anschließend mit durchsichtigen Plastikhauben abgedeckt, bis die Pflanzen angewachsen waren. Diese Abdeckung bewirkt ein gleichmäßiges Keimen der Testpflanzen, sofern dies nicht durch die Wirkstoffe beeinträchtigt wurde.

Zum Zwecke der Nachauflaufbehandlung wurden die Testpflanzen je nach Wuchsform erst bei einer Wuchshöhe von 3 bis 15 cm mit den in Wasser suspendierten oder emulgierten Wirkstoffen behandelt.

Die Pflanzen wurden artenspezifisch bei Temperaturen von 10-25 °C bzw. 20-35 °C gehalten. Die Versuchsperiode erstreckte sich über 2 bis 4 Wochen. Während dieser Zeit wurden die Pflanzen gepflegt und ihre Reaktion auf die einzelnen Behandlungen wurde ausgewertet.

Bewertet wurde nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

## Ansprüche

1. 3-substituierte Pyridine der allgemeinen Formel I

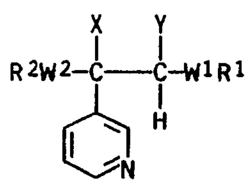

I

in der die Variablen die folgende Bedeutung haben:

X

die Hydroxylgruppe und

y

einen Rest -O-CHO, $-O-CO-W^3R^3$ oder $-O-SO_2-W^3R^3$ oder

X und Y

zusammen Sauerstoff oder eine Gruppe

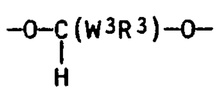 , 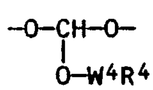 oder 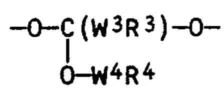 ;

$W^1$-$W^4$

eine direkte Bindung oder eine Gruppe $-CH_2-$, $-CH_2-CH_2-$, $-CH(CH_3)-$, $-CH_2O-$ oder $-CH_2S-$, wobei die Verknüpfung der beiden letzten Gruppen mit den Resten $R^1$ bis $R^4$ über das Sauerstoff- bzw. Schwefelatom erfolgt;

$R^1$-$R^4$

eine $C_1$-$C_6$-Alkylgruppe, die einen $C_3$-$C_8$-Cycloalkylrest tragen kann, eine partiell oder vollständig halogenierte $C_1$-$C_6$-Alkylgruppe, eine $C_3$-$C_8$-Cycloalkylgruppe, die noch bis zu 3 $C_1$-$C_6$-Alkylgruppen tragen kann, eine $C_1$-$C_4$-Alkoxy-$C_1$-$C_4$-alkylgruppe, eine $C_1$-$C_4$-Alkylthio-$C_1$-$C_4$-alkylgruppe, eine $C_2$-$C_6$-Alkenylgruppe, eine $C_2$-$C_6$-Alkinylgruppe, die Phenyl- oder Naphthylgruppe, die beide noch 1 oder 2 der folgenden Reste tragen können: Nitro, Cyano, Halogen, $C_1$-$C_4$-Alkyl, partiell oder vollständig halogeniertes $C_1$-$C_4$-Alkyl, $C_1$-$C_4$-Alkoxy, $C_1$-$C_4$-Alkoxyimino, Phenyl oder Phenoxy mit gewünschtenfalls jeweils 1 bis 5 Halogenatomen am Aromat und wobei die Phenyl- oder Naphthylgruppe noch so viele Halogenatome, $C_1$-$C_4$-Alkylreste, partiell oder vollständig halogenierte $C_1$-$C_4$-Alkylreste und/oder $C_1$-$C_4$-Alkoxyreste tragen kann, daß die Gesamtzahl der Reste 5 beträgt; eine 5- oder 6-gliedrige

Heteroarylgruppe mit einem Stickstoff-, Sauerstoff-oder Schwefelatom und gewünschtenfalls bis zu 2 weiteren Stickstoffatomen als Heteroatome, ausgenommen Heterocyclen mit 3 benachbarten Heteroatomen, wobei der Heteroaromat noch bis zu 3 Halogenatome, $C_1$-$C_4$-Alkylreste, partiell oder vollständig halogenierte $C_1$-$C_4$-Alkylreste oder $C_1$-$C_4$-Alkoxyreste oder bis zu 2 der folgenden Reste tragen kann: Cyano, Nitro, $C_1$-$C_4$-Alkoxyimino, Phenyl oder Phenoxy, die beide am Aromat noch bis zu 5 Halogenatome tragen können;

$R^2$

zusätzlich Wasserstoff, wenn X und Y zusammen einen Rest

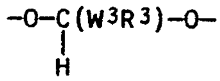 , 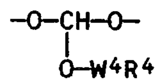 oder 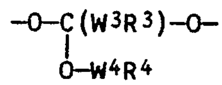 ;

bilden und $W^2$ eine direkte Bindung bedeutet,
ausgenommen cis-1-(2,4-Dichlorphenyl)-2-(pyrid-3-yl)-1,2-epoxypropan, trans-1-(2,4-Dichlorphenyl-2-(pyrid-3-yl)-1,2-epoxypropan, 1-(2,4-Dichlorphenyl)-2-(pyrid-3-yl)-1,2-epoxybutan und Methansulfonsäure-2,4-dichlor-α-[1-hydroxy-1-(pyrid-3-yl)-ethyl]-benzylester,
sowie die N-Oxide und die pflanzenverträglichen mineralsauren Salze und Metallkomplexe von I.

**2.** 3-substituierte Pyridine der Formel I nach Anspruch I, wobei die Variablen die folgende Bedeutung haben:

X

die Hydroxylgruppe und

Y

einen Rest $-O-CO-W^3R^3$ oder $-O-SO_2-W^3R^3$

X und Y

zusammen Sauerstoff oder eine Gruppe

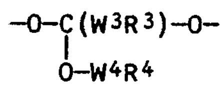 ;

$W^1$-$W^4$ eine direkte Bindung;

$R^1$

2,4-Dichlorphenyl;

$R^2$

$C_1$-$C_4$-Alkyl, Vinyl oder halogeniertes Phenyl;

$R^3$, $R^4$

Methyl.

**3.** 3-substituierte Pyridine der Formel I nach Anspruch I, wobei die Variablen die folgende Bedeutung haben:

X und Y

zusammen eine Gruppe

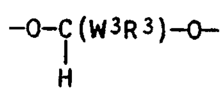

$W^1$-$W^3$

eine direkte Bindung;

$R^1$

2-Bromphenyl, 2-Chlorphenyl oder 2,4-Dichlorphenyl;

$R^2$

Wasserstoff und

$R^3$
Phenyl oder 4-Methylphenyl.

4. Verfahren zur Herstellung der 3-substituierten Pydridine I gemäß Anspruch 1, wobei X eine Hydroxylgruppe und Y einen Rest -O-CHO, -O-CO-$W^3R^3$ oder -O-$SO_2$-$W^3R^3$ bedeuten, dadurch gekennzeichnet, daß man ein Pyridylethandiol der Formel II

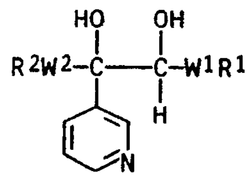 II

in Gegenwart einer Base und gewünschtenfalls eines Katalysators, mit einer Verbindung der Formel IIIa, IIIb oder IIIc

| Z–CHO | Z–CO–$W^3R^3$ | Z–$SO_2$–$W^3R^3$ , |
|---|---|---|
| IIIa | IIIb | IIIc |

wobei Z ein Halogenatom, einen Acyloxyrest oder einen Sulfonyloxyrest bedeutet,
umsetzt und die erhaltenen Verbindungen gewünschtenfalls in die N-Oxide und/oder in ihre Salze oder Metallkomplexe überführt.

5. Verfahren zur Herstellung der 3-substituierten Pyridine I gemäß Anspruch 1, wobei X und Y zusammen Sauerstoff bedeuten, dadurch gekennzeichnet, daß man ein 3-substituiertes Pyridin der Formel Ia

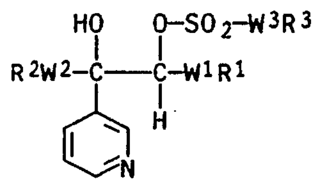 Ia

mit einer Base umsetzt und die erhaltenen Verbindungen gewünschtenfalls in die N-Oxide und/oder in ihre Salze oder Metallkomplexe überführt.

6. Verfahren zur Herstellung der 3-substituierten Pyridine I gemäß Anspruch 1, wobei X und Y zusammen Sauerstoff bedeuten, dadurch gekennzeichnet, daß man ein Pyridylethandiol II in Gegenwart einer Base im Überschuß mit einer Verbindung IIIa, IIIb oder IIIc umsetzt.

7. Verfahren zur Herstellung der 3-substituierten Pydridine I gemäß Anspruch 1, wobei X und Y zusammen eine Gruppe

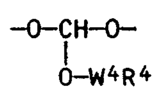 oder 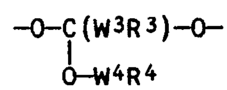

bedeuten, dadurch gekennzeichnet, daß man ein Pyridinethandiol II in Gegenwart einer Säure mit einem Orthoester der Formel IVa oder IVb

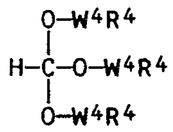 IVa 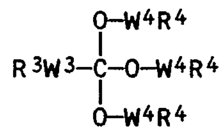 IVb

umsetzt, und die erhaltenen Verbindungen gewünschtenfalls in die N-Oxide und/oder in ihre Salze oder Metallkomplexe überführt.

8. Verfahren zur Herstellung der 3-substituierten Pyridine I gemäß Anspruch 1, wobei X und Y zusammen eine Gruppe

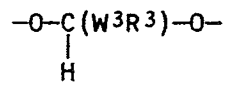

bedeuten dadurch gekennzeichnet, daß man ein Pyridinethandiol II mit einem Aldehyd der Formel V

$$OHC\text{-}W^3R^3 \qquad V$$

oder mit einer difunktionellen Verbindung der Formel VI

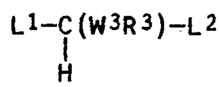 VI

wobei $L^1$ und $L^2$ ein Halogenatom oder eine $C_1$-$C_2$-Alkoxygruppe bedeuten, umsetzt, und die erhaltenen Verbindungen gewünschtenfalls in die N-Oxide und/oder in ihre Salze oder Metallkomplexe überführt.

9. Verwendung der 3-substituierten Pydrine I, deren N-Oxiden und deren pflanzenverträglichen Salzen und Metallkomplexen gemäß Anspruch 1 als Fungizide.

10. Fungizides Mittel, enthaltend einen flüssigen oder festen Trägerstoff und eine fungizid wirksame Menge eines 3-substituierten Pyridins der Formel I, dessen N-Oxid und/oder dessen pflanzenverträgliches Salz oder dessen Metallkomplex gemäß Anspruch 1.

11. Verfahren zur Bekämpfung von Pilzen, dadurch gekennzeichnet, daß man eine fungizid wirksame Menge eines 3-substituierten Pyridins der Formel I, von dessen N-Oxid und/oder von dessen pflanzenverträglichen Salzen oder Metallkomplexen gemäß Anspruch 1, auf Pilze, von Pilzbefall bedrohte Pflanzen, deren Lebensraum oder auf das Saatgut der bedrohten Pflanzen einwirken läßt.

12. Pyridinethandiole der allgemeinen Formel II'

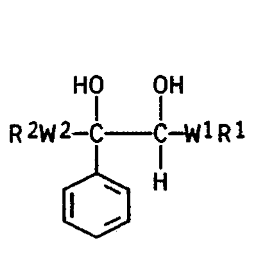 II'

in der die Variablen die folgende Bedeutung haben:
$W^1$;$W^2$
eine direkte Bindung oder eine Gruppe $-CH_2-$, $-CH_2\text{-}CH_2-$, $-CH(CH_3)-$, $-CH_2O-$ oder $-CH_2S-$, wobei die Verknüpfung der beiden letzten Gruppen mit den Resten $R^1$ bis $R^4$ über das Sauerstoff- bzw.

Schwefelatom erfolgt;

$R^1$;$R^2$

eine $C_1$-$C_6$-Alkylgruppe, die einen $C_3$-$C_8$-Cycloalkylrest tragen kann, eine partiell oder vollständig halogenierte $C_1$-$C_6$-Alkylgruppe, eine $C_3$-$C_8$-Cycloalkylgruppe, die noch bis zu 3 $C_1$-$C_6$-Alkylgruppen tragen kann, eine $C_1$-$C_4$-Alkoxy-$C_1$-$C_4$-alkylgruppe, eine $C_1$-$C_4$-Alkylthio-$C_1$-$C_4$-alkylgruppe, eine $C_2$-$C_6$-Alkenylgruppe, eine $C_2$-$C_6$-Alkinylgruppe, die Phenyl- oder Naphthylgruppe, die beide noch 1 oder 2 der folgenden Reste tragen können: Nitro, Cyano, Halogen, $C_1$-$C_4$-Alkyl, partiell oder vollständig halogeniertes $C_1$-$C_4$-Alkyl, $C_1$-$C_4$-Alkoxy, $C_1$-$C_4$-Alkoxyimino, Phenyl oder Phenoxy mit gewünschtenfalls jeweils 1 bis 5 Halogenatomen am Aromat und wobei die Phenyl- oder Naphthylgruppe noch so viele Halogenatome, $C_1$-$C_4$-Alkylreste, partiell oder vollständig halogenierte $C_1$-$C_4$-Alkylreste und/oder $C_1$-$C_4$-Alkoxyreste tragen kann, daß die Gesamtzahl der Reste 5 beträgt; eine 5- oder 6-gliedrige Heteroarylgruppe mit einem Stickstoff-, Sauerstoff-oder Schwefelatom und gewünschtenfalls bis zu 2 weiteren Stickstoffatomen als Heteroatome, ausgenommen Heterocyclen mit 3 benachbarten Heteroatomen, wobei der Heteroaromat noch bis zu 3 Halogenatome, $C_1$-$C_4$-Alkylreste, partiell oder vollständig halogenierte $C_1$-$C_4$-Alkylreste oder $C_1$-$C_4$-Alkoxyreste oder bis zu 2 der folgenden Reste tragen kann: Cyano, Nitro, $C_1$-$C_4$-Alkoxyimino, Phenyl oder Phenoxy, die beide am Aromat noch bis zu 5 Halogenatome tragen können;

mit der Maßgabe, daß, wenn

$R^2W^2$ für Wasserstoff steht, $R^1W^1$ nicht Wasserstoff, Vinyl, Phenyl oder 3-Pyridyl bedeutet,

und der Maßgabe, daß, wenn $R^2W^2$ für Methyl steht, $R^1W^1$ nicht 2,4-Dichlorphenyl bedeutet,

und der Maßgabe, daß, wenn $R^1W^1$ für unsubstituiertes Phenyl steht, $R^2W^2$ nicht unsubstituiertes Phenyl oder 4-Methoxyphenyl bedeuten,

und der Maßgabe, daß, wenn $R^1$ für Wasserstoff oder $C_1$-$C_5$-Alkyl und $R^2$ für gegebenenfalls substituiertes Phenyl und $W^1$ für eine direkte Bindung stehen, $W^2$ nicht -$CH_2$- oder -$CH(CH_3)$- bedeutet.

**13.** Acyloine der allgemeinen Formeln VIIa und VIIb

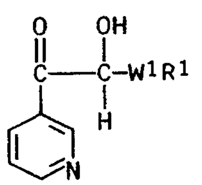

VIIa

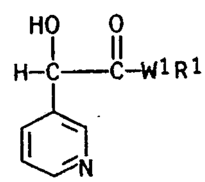

VIIb

in der die Variablen die folgende Bedeutung haben:

$W^1$

eine direkte Bindung oder eine Gruppe -$CH_2$-, -$CH_2$-$CH_2$-, -$CH(CH_3)$-, -$CO_2O$- oder -$CH_2S$-, wobei die Verknüpfung der beiden letzten Gruppen mit den Resten $R^1$ bis $R^4$ über das Sauerstoff- bzw. Schwefelatom erfolgt;

$R^1$

eine $C_1$-$C_6$-Alkylgruppe, die einen $C_3$-$C_8$-Cycloalkylrest tragen kann, eine partiell oder vollständig halogenierte $C_1$-$C_6$-Alkylgruppe, eine $C_3$-$C_8$-Cycloalkylgruppe, die noch bis zu 3 $C_1$-$C_6$-Alkylgruppen tragen kann, eine $C_1$-$C_4$-Alkoxy-$C_1$-$C_4$-alkylgruppe, eine $C_1$-$C_4$-Alkylthio-$C_1$-$C_4$-alkylgruppe. eine $C_2$-$C_6$-Alkenylgruppe, eine $C_2$-$C_6$-Alkinylgruppe, die Phenyl- oder Naphthylgruppe, die beide noch 1 oder 2 der folgenden Reste tragen können: Nitro, Cyano, Halogen, $C_1$-$C_4$-Alkyl, partiell oder vollständig halogeniertes $C_1$-$C_4$-Alkyl, $C_1$-$C_4$-Alkoxy, $C_1$-$C_4$-Alkoxyimino, Phenyl oder Phenoxy mit gewünschtenfalls jeweils 1 bis 5 Halogenatomen am Aromat und wobei die Phenyl- oder Naphthylgruppe noch so viele Halogenatome, $C_1$-$C_4$-Alkylreste, partiell oder vollständig halogenierte $C_1$-$C_4$-Alkylreste und/oder $C_1$-$C_4$-Alkoxyreste tragen kann, daß die Gesamtzahl der Reste 5 beträgt; eine 5- oder 6-gliedrige Heteroarylgruppe mit einem Stickstoff-, Sauerstoff- oder Schwefelatom und gewünschtenfalls bis zu 2 weiteren Stickstoffatomen als Heteroatome, ausgenommen Heterocyclen mit 3 benachbarten Heteroatomen, wobei der Heteroaromat noch bis zu 3 Halogenatome, $C_1$-$C_4$-Alkylreste, partiell oder vollständig halogenierte $C_1$-$C_4$-Alkylreste oder $C_1$-$C_4$-Alkoxyreste oder bis zu 2 der folgenden Reste tragen kann: Cyano, Nitro, $C_1$-$C_4$-Alkoxyimino, Phenyl oder Phenoxy, die beide am Aromat noch bis zu 5 Halogenatome tragen können;

mit der Maßgabe, daß $R^1W^1$ nicht für Wasserstoff, Methyl, unsubstituiertes Phenyl oder 4-Fluorphenyl

steht.

Patentansprüche für folgenden Vertragsstaat:ES

1. Fungizides Mittel, enthaltend einen flüssigen oder festen Trägerstoff und eine fungizid wirksame Menge eines 3-subsitutierten Pyridins der allgemeinen Formel I

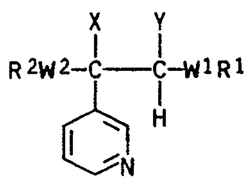 I

in der die Variablen die folgende Bedeutung haben:

X

die Hydroxylgruppe und

Y

einen Rest -O-CHO, $-O-CO-W^3R^3$ oder $-O-SO_2-W^3R^3$ oder

X und Y

zusammen Sauerstoff oder eine Gruppe

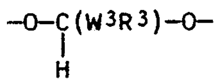 , 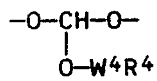 oder 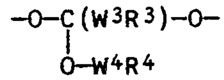 ;

$W^1$-$W^4$

eine direkte Bindung oder eine Gruppe $-CH_2-$, $-CH_2-CH_2-$, $-CH(CH_3)-$, $-CH_2O-$ oder $-CH_2S-$, wobei die Verknüpfung der beiden letzten Gruppen mit den Resten $R^1$ bis $R^4$ über das Sauerstoff- bzw. Schwefelatom erfolgt;

$R^1$-$R^4$

eine $C_1$-$C_6$-Alkylgruppe, die einen $C_3$-$C_8$-Cycloalkylrest tragen kann, eine partiell oder vollständig halogenierte $C_1$-$C_6$-Alkylgruppe, eine $C_3$-$C_8$-Cycloalkylgruppe, die noch bis zu 3 $C_1$-$C_6$-Alkylgruppen tragen kann, eine $C_1$-$C_4$-Alkoxy-$C_1$-$C_4$-alkylgruppe, eine $C_1$-$C_4$-Alkylthio-$C_1$-$C_4$-alkylgruppe, eine $C_2$-$C_6$-Alkenylgruppe, eine $C_2$-$C_6$-Alkinylgruppe, die Phenyl- oder Naphthylgruppe, die beide noch 1 oder 2 der folgenden Reste tragen können: Nitro, Cyano, Halogen, $C_1$-$C_4$-Alkyl, partiell oder vollständig halogeniertes $C_1$-$C_4$-Alkyl, $C_1$-$C_4$-Alkoxy, $C_1$-$C_4$-Alkoxyimino, Phenyl oder Phenoxy mit gewünschtenfalls jeweils 1 bis 5 Halogenatomen am Aromat und wobei die Phenyl- oder Naphthylgruppe noch so viele Halogenatome, $C_1$-$C_4$-Alkylreste, partiell oder vollständig halogenierte $C_1$-$C_4$-Alkylreste und/oder $C_1$-$C_4$-Alkoxyreste tragen kann, daß die Gesamtzahl der Reste 5 beträgt; eine 5- oder 6-gliedrige Heteroarylgruppe mit einem Stickstoff-, Sauerstoff-oder Schwefelatom und gewünschtenfalls bis zu 2 weiteren Stickstoffatomen als Heteroatome, ausgenommen Heterocyclen mit 3 benachbarten Heteroatomen, wobei der Heteroaromat noch bis zu 3 Halogenatome, $C_1$-$C_4$-Alkylreste, partiell oder vollständig halogenierte $C_1$-$C_4$-Alkylreste oder $C_1$-$C_4$-Alkoxyreste oder bis zu 2 der folgenden Reste tragen kann: Cyano, Nitro, $C_1$-$C_4$-Alkoxyimino, Phenyl oder Phenoxy, die beide am Aromat noch bis zu 5 Halogenatome tragen können;

$R^2$

zusätzlich Wasserstoff, wenn X und Y zusammen einen Rest

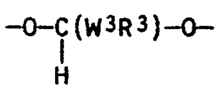 , 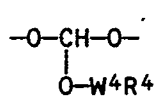 oder 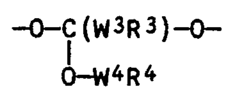 ;

bilden und $W^2$ eine direkte Bindung bedeutet,

ausgenommen cis-1-(2,4-Dichlorphenyl)-2-(pyrid-3-yl)-1,2-epoxypropan, trans-1-(2,4-Dichlorphenyl)-2-

(pyrid-3-yl)-1,2-epoxypropan, 1-(2,4-Dichlorphenyl)-2-(pyrid-3-yl)-1,2-epoxybutan und Methansulfonsäure-2,4-dichlor-α-[1-hydroxy-1-(pyrid-3-yl)-ethyl]-benzylester, dessen N-Oxid und/oder dessen pflanzenverträgliches Salz oder dessen Metallkomplex.

**2.** Fungizides Mittel, enthaltend einen flüssigen oder festen Trägerstoff und eine fungikzid wirksame Menge eines 3-substituierten Pyridins der Formel I nach Anspruch 1, wobei die Variablen die folgende Bedeutung haben:
X
die Hydroxylgruppe und
Y
einen Rest -O-CO-$W^3R^3$ oder -O-$SO_2$-$W^3R^3$
X und Y
zusammen Sauerstoff oder eine Gruppe

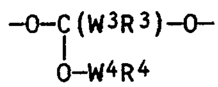 ;

$W^1$ - $W^4$ eine direkte Bindung;
$R^1$
2,4-Dichlorphenyl;
$R^2$
$C_1$-$C_4$-Alkyl, Vinyl oder halogeniertes Phenyl;
$R^3$, $R^4$
Methyl.

**3.** Fungizides Mittel, enthaltend einen flüssigen oder festen Trägerstoff und eine fungizid wirksame Menge eines 3-substituiertes Pyridins der Formel I nach Anspruch 1, wobei die Variablen die folgende Bedeutung haben: X und Y
zusammen eine Gruppe

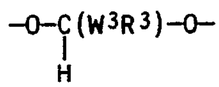

$W^1$-$W^3$
eine direkte Bindung;
$R^1$
2-Bromphenyl, 2-Chlorphenyl oder 2,4-Dichlorphenyl;
$R^2$
Wasserstoff und
$R^3$
Phenyl oder 4-Methylphenyl.

**4.** Verfahren zur Herstellung der 3-substituierten Pydridine I gemäß Anspruch 1, wobei X eine Hydroxylgruppe und Y einen Rest -O-CHO, -O-CO-$W^3R^3$ oder -O-$SO_2$-$W^3R^3$ bedeuten, dadurch gekennzeichnet, daß man ein Pyridylethandiol der Formel II

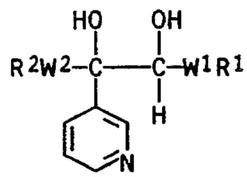

II

in Gegenwart einer Base und gewünschtenfalls eines Katalysators, mit einer Verbindung der Formel IIIa, IIIb oder IIIc

| | | |
|---|---|---|
| Z–CHO | Z–CO–$W^3R^3$ | Z–$SO_2$–$W^3R^3$ , |
| IIIa | IIIb | IIIc |

wobei Z ein Halogenatom, einen Acyloxyrest oder einen Sulfonyloxyrest bedeutet, umsetzt und die erhaltenen Verbindungen gewünschtenfalls in die N-Oxide und/oder in ihre Salze oder Metallkomplexe überführt.

5. Verfahren zur Herstellung der 3-substituierten Pyridine I gemäß Anspruch 1, wobei X und Y zusammen Sauerstoff bedeuten, dadurch gekennzeichnet, daß man ein 3-substituiertes Pyridin der Formel Ia

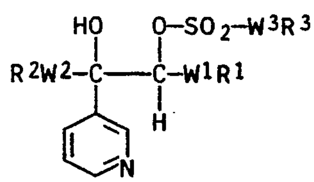

Ia

mit einer Base umsetzt und die erhaltenen Verbindungen gewünschtenfalls in die N-Oxide und/oder in ihre Salze oder Metallkomplexe überführt.

6. Verfahren zur Herstellung der 3-substituierten Pyridine I gemäß Anspruch 1, wobei X und Y zusammen Sauerstoff bedeuten, dadurch gekennzeichnet, daß man ein Pyridylethandiol II in Gegenwart einer Base im Überschuß mit einer Verbindung IIIa, IIIb oder IIIc umsetzt.

7. Verfahren zur Herstellung der 3-substituierten Pydridine I gemäß Anspruch 1, wobei X und Y zusammen eine Gruppe

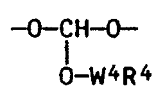

oder

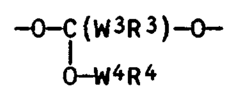

bedeuten, dadurch gekennzeichnet, daß man ein Pyridinethandiol II in Gegenwart einer Säure mit einem Orthoester der Formel IVa oder IVb

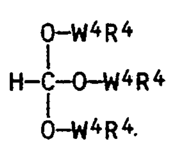

IVa

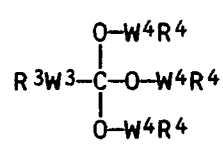

IVb

umsetzt, und die erhaltenen Verbindungen gewünschtenfalls in die N-Oxide und/oder in ihre Salze oder Metallkomplexe überführt.

8. Verfahren zur Herstellung der 3-substituierten Pyridine I gemäß Anspruch 1, wobei X und Y zusammen eine Gruppe

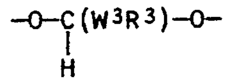

bedeuten dadurch gekennzeichnet, daß man ein Pyridinethandiol II mit einem Aldehyd der Formel V

$$OHC-W^3R^3 \qquad V$$

oder mit einer difunktionellen Verbindung der Formel VI

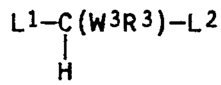 VI

wobei $L^1$ und $L^2$ ein Halogenatom oder eine $C_1$-$C_2$-Alkoxygruppe bedeuten, umsetzt, und die erhaltenen Verbindungen gewünschtenfalls in die N-Oxide und/oder in ihre Salze oder Metallkomplexe überführt.

9. Verfahren zur Bekämpfung von Pilzen, dadurch gekennzeichnet, daß man eine fungizid wirksame Menge eines 3-substituierten Pyridins der Formel I, von dessen N-Oxid und/oder von dessen pflanzenverträglichen Salzen oder Metallkomplexen gemäß Anspruch 1, auf Pilze, von Pilzbefall bedrohte Pflanzen, deren Lebensraum oder auf das Saatgut der bedrohten Pflanzen einwirken läßt.

10. Verfahren zur Herstellung von Pyridinethandiolen der allgemeinen Formel II'

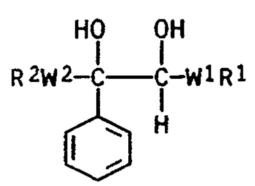 II'

in der die Variablen die folgende Bedeutung haben:
$W^1$;$W^2$
eine direkte Bindung oder eine Gruppe $-CH_2-$, $-CH_2-CH_2-$, $-CH(CH_3)-$, $-CH_2O-$ oder $-CH_2S-$, wobei die Verknüpfung der beiden letzten Gruppen mit den Resten $R^1$ bis $R^4$ über das Sauerstoff- bzw. Schwefelatom erfolgt;
$R^1$;$R^2$
eine $C_1$-$C_6$-Alkylgruppe, die einen $C_3$-$C_8$-Cycloalkylrest tragen kann, eine partiell oder vollständig halogenierte $C_1$-$C_6$-Alkylgruppe, eine $C_3$-$C_8$-Cycloalkylgruppe, die noch bis zu 3 $C_1$-$C_6$-Alkylgruppen tragen kann, eine $C_1$-$C_4$-Alkoxy-$C_1$-$C_4$-alkylgruppe, eine $C_1$-$C_4$-Alkylthio-$C_1$-$C_4$-alkylgruppe, eine $C_2$-$C_6$-Alkenylgruppe, eine $C_2$-$C_6$-Alkinylgruppe, die Phenyl- oder Naphthylgruppe, die beide noch 1 oder 2 der folgenden Reste tragen können: Nitro, Cyano, Halogen, $C_1$-$C_4$-Alkyl, partiell oder vollständig halogeniertes $C_1$-$C_4$-Alkyl, $C_1$-$C_4$-Alkoxy, $C_1$-$C_4$-Alkoxyimino, Phenyl oder Phenoxy mit gewünschtenfalls jeweils 1 bis 5 Halogenatomen am Aromat und wobei die Phenyl- oder Naphthylgruppe noch so viele Halogenatome, $C_1$-$C_4$-Alkylreste, partiell oder vollständig halogenierte $C_1$-$C_4$-Alkylreste und/oder $C_1$-$C_4$-Alkoxyreste tragen kann, daß die Gesamtzahl der Reste 5 beträgt; eine 5- oder 6-gliedrige Heteroarylgruppe mit einem Stickstoff-, Sauerstoff-oder Schwefelatom und gewünschtenfalls bis zu 2 weiteren Stickstoffatomen als Heteroatome, ausgenommen Heterocyclen mit 3 benachbarten Heteroatomen, wobei der Heteroaromat noch bis zu 3 Halogenatome, $C_1$-$C_4$-Alkylreste, partiell oder vollständig halogenierte $C_1$-$C_4$-Alkylreste oder $C_1$-$C_4$-Alkoxyreste oder bis zu 2 der folgenden Reste tragen kann: Cyano, Nitro, $C_1$-$C_4$-Alkoxyimino, Phenyl oder Phenoxy, die beide am Aromat noch bis zu 5 Halogenatome tragen können;
mit der Maßgabe, daß, wenn
$R^2W^2$ für Wasserstoff steht, $R^1W^1$ nicht Wasserstoff, Vinyl, Phenyl oder 3-Pyridyl bedeutet,
und der Maßgabe, daß, wenn $R^2W^2$ für Methyl steht, $R^1W^1$ nicht
2,4-Dichlorphenyl bedeutet,
und der Maßgabe, daß, wenn $R^1W^1$ für unsubstituiertes Phenyl steht, $R^2W^2$ nicht unsubstituiertes Phenyl oder 4-Methoxyphenyl bedeuten,
und der Maßgabe, daß, wenn $R^1$ für Wasserstoff oder $C_1$-$C_5$-Alkyl und $R^2$ für gegebenenfalls substituiertes Phenyl und $W^1$ für eine direkte Bindung stehen, $W^2$ nicht $-CH_2-$ oder $-CH(CH_3)-$ bedeutet,
dadurch gekennzeichnet, daß man

a) Acyloine der Formel VIIa oder VIIb

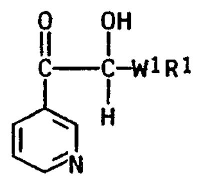

VIIa

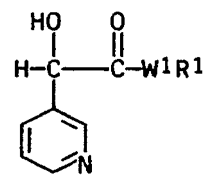

VIIb

reduziert, oder
b) eine Organometallverbindung der Formel VIII

$$R^2W^2-M \qquad VIII$$

an ein Acyloin der Formel VIIa oder VIIa'

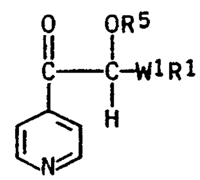

VIIa'

wobei $R^5$ für eine Schutzgruppe der OH-Funktion steht, addiert oder
c) ein Pyridylketon der Formel IX

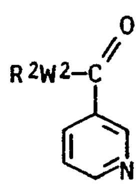

IX

mit Dimethylsulfonium- oder Diethylsulfoxoniummethylid in ein Oxiran der Formel X

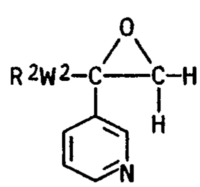

X

überführt und dieses unter Säure- oder Basenkatalyse hydrolysiert.

**11.** Verfahren zur Herstellung von Acyloinen der allgemeinen Formeln VIIa und VIIb

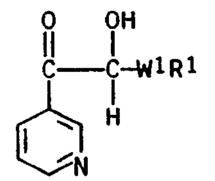

VIIa

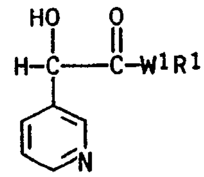

VIIb

in der die Variablen die folgende Bedeutung haben: $W^1$

eine direkte Bindung oder eine Gruppe $-CH_2-$, $-CH_2-CH_2-$, $-CH(CH_3)-$, $-CH_2O-$ oder $-CH_2S-$, wobei die Verknüpfung der beiden letzten Gruppen mit den Resten $R^1$ bis $R^4$ über das Sauerstoff- bzw. Schwefelatom erfolgt;

$R^1$

eine $C_1$-$C_6$-Alkylgruppe, die einen $C_3$-$C_8$-Cycloalkylrest tragen kann, eine partiell oder vollständig halogenierte $C_1$-$C_6$-Alkylgruppe, eine $C_3$-$C_8$-Cycloalkylgruppe, die noch bis zu 3 $C_1$-$C_6$-Alkylgruppen tragen kann, eine $C_1$-$C_4$-Alkoxy-$C_1$-$C_4$-alkylgruppe, eine $C_1$-$C_4$-Alkylthio-$C_1$-$C_4$-alkylgruppe, eine $C_2$-$C_6$-Alkenylgruppe, eine $C_2$-$C_6$-Alkinylgruppe, die Phenyl- oder Naphthylgruppe, die beide noch 1 oder 2 der folgenden Reste tragen können: Nitro, Cyano, Halogen, $C_1$-$C_4$-Alkyl, partiell oder vollständig halogeniertes $C_1$-$C_4$-Alkyl, $C_1$-$C_4$-Alkoxy, $C_1$-$C_4$-Alkoxyimino, Phenyl oder Phenoxy mit gewünschtenfalls jeweils 1 bis 5 Halogenatomen am Aromat und wobei die Phenyl- oder Naphthylgruppe noch so viele Halogenatome, $C_1$-$C_4$-Alkylreste, partiell oder vollständig halogenierte $C_1$-$C_4$-Alkylreste und/oder $C_1$-$C_4$-Alkoxyreste tragen kann, daß die Gesamtzahl der Reste 5 beträgt; eine 5- oder 6-gliedrige Heteroarylgruppe mit einem Stickstoff-, Sauerstoff- oder Schwefelatom und gewünschtenfalls bis zu 2 weiteren Stickstoffatomen als Heteroatome, ausgenommen Heterocyclen mit 3 benachbarten Heteroatomen, wobei der Heteroaromat noch bis zu 3 Halogenatome, $C_1$-$C_4$-Alkylreste, partiell oder vollständig halogenierte $C_1$-$C_4$-Alkylreste oder $C_1$-$C_4$-Alkoxyreste oder bis zu 2 der folgenden Reste tragen kann: Cyano, Nitro, $C_1$-$C_4$-Alkoxyimino, Phenyl oder Phenoxy, die beide am Aromat noch bis zu 5 Halogenatome tragen können;

mit der Maßgabe, daß $R^1W^1$ nicht für Wasserstoff, Methyl, unsubstituiertes Phenyl oder 4-Fluorphenyl steht,

dadurch gekennzeichnet, daß man 3-Pyridylaldehyd, gewünschtenfalls in Gegenwart eines Katalysators, mit Aldehyden der Formel XI

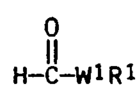

XI

kondensiert.